(19) **Europäisches Patentamt / European Patent Office / Office européen des brevets**

(11) **EP 3 709 866 B1**

(12) **FASCICULE DE BREVET EUROPEEN**

(45) Date de publication et mention de la délivrance du brevet:
**05.07.2023 Bulletin 2023/27**

(21) Numéro de dépôt: **18821717.8**

(22) Date de dépôt: **16.11.2018**

(51) Classification Internationale des Brevets (IPC):
***A61B 5/00*** *(2006.01)*

(52) Classification Coopérative des Brevets (CPC):
**A61B 5/4023**

(86) Numéro de dépôt international:
**PCT/FR2018/052895**

(87) Numéro de publication internationale:
**WO 2019/097188 (23.05.2019 Gazette 2019/21)**

(54) **PROCÉDÉ AMÉLIORÉ DE QUANTIFICATION DE L'ÉQUILIBRE**

VERBESSERTES VERFAHREN ZUR QUANTIFIZIERUNG DES GLEICHGEWICHTS

IMPROVED METHOD FOR QUANTIFYING BALANCE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.11.2017 FR 1760888**

(43) Date de publication de la demande:
**23.09.2020 Bulletin 2020/39**

(73) Titulaires:
- **Assistance Publique - Hôpitaux de Paris**
  **75004 Paris (FR)**
- **École Normale Supérieure Paris-Saclay**
  **94230 Cachan (FR)**
- **Univ Paris XIII Paris-Nord Villetaneuse**
  **93430 Villetaneuse (FR)**
- **Etat Français - Ministère de la Défense - Direction centrale du service de santé des armées**
  **75614 Paris Cedex 12 (FR)**
- **Centre National de la Recherche Scientifique**
  **75016 Paris (FR)**
- **Université Paris Cité**
  **75006 Paris (FR)**

(72) Inventeurs:
- **BARGIOTAS, Ioannis**
  **75011 Paris (FR)**
- **AUDIFFREN, Julien**
  **1700 Fribourg (CH)**
- **OUDRE, Laurent**
  **75012 Paris (FR)**
- **BUFFAT, Stéphane**
  **75016 Paris (FR)**
- **VAYATIS, Nicolas**
  **75005 Paris (FR)**
- **VIDAL, Pierre Paul**
  **75005 Paris (FR)**
- **RICARD, Damien**
  **92140 Clamart (FR)**
- **YELNIK, Alain**
  **92240 Malakoff (FR)**

(74) Mandataire: **A.P.I. Conseil**
**Immeuble Newton**
**4, rue Jules Ferry**
**64000 Pau (FR)**

(56) Documents cités:
**US-A- 5 388 591**     **US-A1- 2016 007 902**
**US-A1- 2017 000 387**

- **JULIEN AUDIFFREN ET AL: "A Non Linear Scoring Approach for Evaluating Balance: Classification of Elderly as Fallers and Non-Fallers", PLOS ONE, vol. 11, no. 12, 9 décembre 2016 (2016-12-09), page e0167456, XP055501700, DOI: 10.1371/journal.pone.0167456**

## Description

La présente invention concerne le domaine de la quantification de l'équilibre d'un individu. La présente invention concerne plus particulièrement un procédé pour la quantification de l'équilibre d'une personne, un dispositif apte à mettre en oeuvre ce procédé et un système intégrant ledit dispositif. La présente invention permet en particulier un suivi de l'évolution de cet équilibre notamment de façon à alerter sur des risques de chute, par exemple dans le cadre d'un processus de rééducation, dans une démarche de quantification de soi ou chez la personne âgée.

## [Art antérieur]

Les équilibres statiques et dynamiques sont des composantes essentielles de nos déplacements de tous les jours et un défaut d'équilibre est une cause majeure de chute. Le contrôle postural est obtenu grâce à la combinaison de systèmes visuels, proprioceptifs et vestibulaires, ainsi que du système nerveux central. Des déficiences ou des troubles de ces systèmes peuvent détériorer progressivement le contrôle postural de l'individu et augmenteront par conséquent le risque de chute.

Selon des estimations de l'Organisation Mondiale de la Santé de 2012, près de 424 000 personnes dans le monde perdent la vie chaque année à la suite de chutes, plaçant ainsi les chutes à la deuxième place des causes de décès accidentels dans le monde. Selon l'Institut de Veille Sanitaire, il y a chaque année en France 450 000 chutes chez les personnes âgées de plus de 65 ans et c'est la cause la plus fréquente de décès chez les personnes âgées avec 4000 à 4500 cas par an en France.

La chute des personnes est donc un problème majeur de santé publique en raison de sa fréquence et de ses conséquences médicales et sociales ; notamment et surtout chez l'individu âgé. En effet, de nos jours, les chutes sont considérées comme l'une des principales causes de blessures chez les personnes âgées et, si elles n'entrainent pas la mort, elles peuvent entrainer une réduction accrue de la mobilité et/ou de l'autonomie dans les activités quotidiennes. Par exemple, le syndrome post chute se traduit par une phobie de la chute avec une perte de confiance en soi pour accomplir des actes de la vie quotidienne et conduit à terme, à une grabatisation.

Par conséquent, l'évaluation précise des risques est devenue une question importante, étant donné qu'un tiers de la population âgée (i.e âge supérieur à 65 ans) est confronté à au moins une chute par an. Malgré ces enjeux, il n'existe pas aujourd'hui de procédé ou de dispositif intuitif, fiable et peu coûteux permettant de quantifier l'équilibre d'un individu. Les médecins apprécient aujourd'hui l'équilibre via des méthodes de suivi visuel du patient par exemple au cours de tests normalisés tels que le test de Romberg. Ce dernier peut aider le médecin à poser un diagnostic et identifier les causes possibles d'une ataxie statique. Néanmoins, un tel suivi permet de qualifier et non de quantifier de façon objective l'équilibre d'un individu. Or, des méthodes quantitatives pourraient permettre de renforcer la sensibilité, l'objectivité et l'homogénéité des interprétations, et donneraient la possibilité de faire des comparaisons de tels tests (e.g. suivi dans le temps ou au sein d'un groupe d'individus) ou d'identifier des comportements imperceptibles via des méthodes de suivi visuel du patient.

Dans ce contexte, l'utilisation des plateformes de force pour quantifier et évaluer le contrôle postural se généralise. De telles plateformes enregistrent le déplacement du centre de pression (« center of pressure » - COP, en terminologie Anglo-saxonne) qui est appliqué par l'ensemble du corps dans le temps alors que l'individu se tient sur la plateforme. Cette mesure permet de générer un statokinésigramme. Des indices empiriques dérivés du déplacement du COP ont été proposés précédemment tels que la longueur du déplacement du COP ou la variance de la vitesse de déplacement du COP. Ces indices ont montré que les statokinésigrammes peuvent être significativement affectés par le contrôle postural des individus. Comme exposé dans le document US5388591, le statokinésigramme peut subir par la suite un traitement temporel de ses données afin de générer un graphique de diffusion du statokinésigramme mettant en avant des indices afin d'évaluer la stabilité posturale d'un sujet. En outre, il a été proposé que la distribution d'énergie des bandes de fréquences spécifiques puisse informer les stratégies posturales choisies. Conformément à ce qui précède, des analyses antérieures ont appliqué aux statokinésigrammes une analyse de type transformée de Fourier à court terme (STFT - « short-term Fourier transform » en terminologie Anglo-saxonne) ou une analyse temporelle en ondelettes (« time-scale wavelet analysis » en terminologie Anglo-saxonne) afin d'évaluer les transitions entre les stratégies posturales. La STFT permet d'analyser les changements temporels dans le contenu spectral du déplacement du COP (Schumann et al., 1995. Time-frequency analysis of postural sway. J. Biomechanics, Vol. 28, No. 5, pp. 603-607). Des travaux sur l'analyse temporelle en ondelettes ont montré l'existence d'un lien entre les bandes de fréquences et les principales entrées sensorielles : la bande de basse fréquence (<0,1 Hz) est associée au contrôle visuel, des fréquences comprises entre 0,1 et 0,5 Hz sont dominés par l'activité vestibulaire tandis que la bande de fréquence de 0,5-1 Hz reflète l'activité somatosensorielle. Des études basées sur ce type d'analyse ont notamment conclu que pour l'évaluation des données posturographiques, il est préférable de réaliser des essais d'au moins 60 s, sinon des interprétations erronées peuvent se produire en raison de mauvaises estimations ou même d'extraits biaisés d'un processus d'ensemble (Kirchner et al. 2012, Evaluation of the temporal structure of postural sway fluctuations based on a comprehensive set of analysis tools. Physica A 391 (2012) 4692-4703). De plus, la seule information obtenue lors de la mise en oeuvre de ces méthodes porte sur la distribution de l'énergie en bande de fréquence et l'utilisateur n'est plus en mesure d'apprécier des paramètres bien établis tels que l'amplitude des déplacements suivant les axes antéro-postérieurs et médio-latéral ou encore la surface de déplacement. Des travaux récents ont utilisé la combinaison de paramètres, dans des analyses multiparamétriques de statokinésigrammes, afin d'identifier les patients susceptibles de chuter par rapport à des non-chuteurs (Audiffren et al., 2016, A non linear scoring approach for evaluating balance : classification of elderly as fallers and non-fallers. PLoS ONE 11 (12) - 9 décembre 2016). En particulier, ces travaux divulguent un procédé selon le préambule de la revendication 1. Lorsque des méthodes de quantification ont été utilisées, ces dernières n'ont pas été en mesure de produire des résultats suffisamment satisfaisants car les données à étudier sont très complexes et difficiles à modéliser. Plusieurs raisons constituent un frein à son utilisation : coût d'acquisition, de réalisation, reproductibilité des examens, sensibilité, spécificité, et difficultés d'interprétation des résultats.

Ainsi, les procédés d'analyse utilisés précédemment pour évaluer l'équilibre ne fournissent pas une valeur unique et fiable, mesurable facilement à l'aide de dispositif simple et peu coûteux. Il existe donc un besoin pour un dispositif pour la quantification de l'équilibre qui puisse être utilisé par toute personne soucieuse de suivre son équilibre et cela à faible cout, mais également par les pouvoirs publics ou le personnel de santé.

[**Problème technique**]

L'invention a pour but de remédier aux inconvénients de l'art antérieur. En particulier, l'invention a pour but de proposer un procédé de quantification de l'équilibre fiable, c'est-à-dire permettant d'établir une valeur représentative de l'état de l'équilibre de l'individu, rapide, simple, et ne nécessitant pas nécessairement l'intervention d'un spécialiste dans le domaine de la posturologie. A noter que ce procédé ne vise pas à se substituer au médecin généraliste ou au spécialiste et ne pose pas un diagnostic. Le procédé doit en outre être capable d'identifier des signaux faibles contenus dans les statokinésigrammes de façon à augmenter sa fiabilité.

L'invention a en outre pour but de proposer un dispositif de quantification de l'équilibre pouvant être intégré dans un système complet de quantification de l'équilibre.

[**Brève description de l'invention**]

A cet effet, l'invention porte sur un procédé de quantification de l'équilibre d'un individu pour obtenir une valeur représentative de l'équilibre dudit individu, ledit procédé étant mis en oeuvre par un dispositif comprenant au moins un module de traitement de données connecté à un moyen de mémorisation, ledit procédé comprenant un enregistrement (10), sur le moyen de mémorisation (280), d'au moins un statokinésigramme de l'individu (110) obtenu à partir d'une plateforme (310) comprenant des capteurs (312) de pression et/ou de force, ledit procédé étant caractérisé en ce qu'il comprend en outre les étapes suivantes :

- segmentation en fonction du temps, du ou des statokinésigramme(s) de l'individu enregistré(s) sur le moyen de mémorisation, par le module de traitement de données, de façon à générer plusieurs portions de statokinésigramme(s),
- extraction, par le module de traitement de données et à partir des portions de statokinésigramme(s), des valeurs d'au moins un paramètre de trajectoire, ledit paramètre de trajectoire étant de préférence un paramètre de trajectoire de position, de stabilité et/ou de dynamique,
- détermination, par le module de traitement de données, de la valeur d'au moins deux quantificateurs, à partir des valeurs de paramètres de trajectoire extraites à l'étape d'extraction pour chacune des portions de statokinésigramme(s) générées à l'étape de segmentation,
- détermination, par le module de traitement de données, de ladite valeur représentative de l'équilibre de l'individu à partir des valeurs des quantificateurs de chacune des portions de statokinésigramme(s).

Les statokinésigrammes peuvent présenter des dynamiques de déplacement du centre de pression variables au cours de leur enregistrement. Les approches antérieures, par exemple les approches multidimensionnelles, ont tenté de caractériser globalement les statokinésigrammes. Une telle approche repose notamment sur l'hypothèse implicite selon laquelle le signal peut présenter un profil uniforme. Par conséquent, bien que ces dernières méthodes aient présenté une utilité majeure, elles présentent l'inconvénient d'être sensibles à la non uniformité des statokinésigrammes et à l'existence de périodes de courtes durées associées à un déséquilibre postural transitoire. Ces déséquilibres transitoires peuvent passer inaperçus lors d'une analyse globale de statokinésigrammes, car leurs durées sont généralement de plusieurs dizaines de secondes.

La segmentation permet d'analyser chaque portion indépendamment des autres portions de statokinésigramme de façon à identifier des signaux faibles de déficit de l'équilibre chez un individu. En effet, un épisode de trouble de l'équilibre sur une durée de quelques secondes pourrait être suffisant à une personne pour tomber mais pourrait ne pas être identifié lors de l'analyse d'un statokinésigramme de plusieurs dizaines de secondes pris dans son intégralité. La mise en oeuvre de ce procédé inclut la détermination de plusieurs quantificateurs pour une même portion étant ensuite traités conjointement dans une étape de détermination d'une valeur représentative de l'équilibre.

Selon d'autres caractéristiques optionnelles du procédé :

- les portions de statokinésigramme présentent une durée inférieure ou égale à trois secondes. En effet, les inventeurs ont déterminé que lorsque le statokinésigramme est segmenté en portions de trois secondes ou moins, alors la fiabilité du procédé est améliorée ;
- l'étape de segmentation génère, pour chaque statokinésigramme, au moins dix portions de statokinésigramme. Une pluralité de portions étudiées lors de l'étape de détermination de la valeur représentative de l'équilibre permet d'améliorer la fiabilité du procédé ;
- les portions de statokinésigramme générées lors de l'étape de segmentation présentent, pour des portions consécutives, un taux de recouvrement d'au moins 25 %, de préférence d'au moins 50 %. Comme cela sera présenté dans les exemples et bien que cela puisse augmenter la durée de traitement et de détermination, les inventeurs ont déterminé que lorsque les portions présentent entre elles un recouvrement, alors la fiabilité du procédé est améliorée ;
- les portions de statokinésigramme générées lors de l'étape de segmentation présentent, pour des portions consécutives, un taux de recouvrement d'au plus 95 %.
- l'étape de détermination est réalisée en mettant en oeuvre les valeurs des quantificateurs déterminées à l'étape de détermination dans un algorithme de notation de façon, en fonction des valeurs des quantificateurs, à attribuer un score à chacune des portions de statokinésigrammes ou à classer chacune des portions de statokinésigrammes par catégorie. Le score peut par exemple être un score représentatif de d'un caractéristique du déplacement du centre de pression (régulier ou irrégulier) ou encore être représentatif de la qualité de l'équilibre de l'individu sur chaque portion du statokinésigramme. Alternativement, le procédé peut permettre d'associer chaque portion à une catégorie telle que « équilibre bon » ou « équilibre à risque » ;
- il comprend en outre une étape de transmission, sur un moyen de visualisation, du score ou de la catégorie de chacune des portions de statokinésigramme de façon à permettre la génération d'une représentation graphique des portions du statokinésigramme en fonction dudit score ou de ladite catégorie. Une telle étape permet à un utilisateur de directement identifier les portions de statokinésigrammes pouvant présenter un épisode de déséquilibre postural ou un épisode caractéristique d'un risque de trouble de l'équilibre. Au-delà d'un score global, l'utilisateur peut alors traiter individuellement chacune des portions de façon à améliorer la quantification et la qualification de l'équilibre ;
- l'algorithme de notation est un algorithme de partitionnement non supervisé. Ainsi, le procédé peut être mis en oeuvre sans recours à une base de données ;
- l'algorithme de partitionnement non supervisé est sélectionné parmi un modèle de mélange Gaussien non supervisé, une classification ascendante hiérarchique, une classification descendante hiérarchique. C'est avantageusement un modèle de mélange Gaussien non supervisé ;
- l'algorithme de notation est préalablement calibré sur la base des valeurs des mêmes quantificateurs obtenus à partir de portions de statokinésigrammes de référence. C'est alors de préférence un algorithme d'apprentissage supervisé ;
- il comprend en outre une étape de génération de données brutes correspondant au déplacement du centre d'une pression appliquée par l'ensemble du corps d'un individu dans le temps sur une plateforme. Cela correspond au déplacement du centre de pression ;
- il comprend en outre une étape de transformation des données brutes en données de trajectoire du centre de pression ;
- les données brutes correspondant au déplacement du centre de pression d'un individu sont obtenues lors d'un test de Romberg ;
- l'étape de segmentation est réalisée à partir d'un statokinésigramme obtenu alors que l'individu a les yeux ouverts et d'un statokinésigramme obtenu lorsque l'individu a les yeux fermés ;
- il comporte, pour au moins un quantificateur, le calcul d'un rapport O/F ou F/O correspondant au rapport entre la valeur d'un paramètre de trajectoire calculé à partir d'un statokinésigramme obtenu alors que l'individu a les yeux ouverts et la valeur d'un paramètre de trajectoire calculé à partir d'un statokinésigramme obtenu alors que l'individu a les yeux fermés (rapport O/F) ou l'inverse (rapport F/O) ; et/ou
- une durée d'acquisition d'un statokinésigramme peut être comprise entre 5 et 70 secondes.
- le paramètre de trajectoire est un paramètre de trajectoire de position du centre de pression, de stabilité du centre de pression, et/ou dynamique du centre de pression.

L'invention porte en outre sur un dispositif de quantification de l'équilibre d'un individu, ledit dispositif comprenant :

- un module de communication apte à recevoir un statokinésigramme dudit individu,
- un moyen de mémorisation apte à enregistrer ledit statokinésigramme, et
- au moins un module de traitement de données, apte à se connecter au moyen de mémorisation,

caractérisé en ce que ledit module de traitement de données est configuré pour :

- o Segmenter en fonction du temps le statokinésigramme de l'individu enregistré sur le moyen de mémorisation de façon à générer plusieurs portions de statokinésigramme,
- o Extraire, à partir des portions de statokinésigramme, des valeurs d'au moins un paramètre de trajectoire,
- o Déterminer au moins deux quantificateurs, à partir des valeurs de paramètre de trajectoire extraites,
- o Déterminer une valeur représentative de l'équilibre de l'individu à partir des valeurs desdits quantificateurs de chacune des portions de statokinésigrammes.

**Selon d'autres caractéristiques optionnelles du dispositif :**

- le module de communication est configuré pour recevoir et transmettre des informations à des systèmes distants ;
- il comporte en outre un module de génération de statokinésigramme configuré pour générer les données relatives à un statokinésigramme ; et/ou
- il comporte en outre un module de ré-échantillonnage configuré pour traiter les données brutes ou les statokinésigramme à une première fréquence de façon à générer des statokinésigrammes ré-échantillonnés à une seconde fréquence et présentant une fréquence sensiblement constante.

L'invention porte en outre sur un système de quantification de l'équilibre d'un individu, comprenant :

- une plateforme, ladite plateforme étant adaptée à recevoir un individu et comprenant des capteurs de pression et/ou de force configurés pour générer des données brutes, à une première fréquence, fonction d'une pression exercée par les pieds de l'individu sur la plateforme,
- une unité de traitement des données brutes, agencée pour obtenir au moins un statokinésigramme de l'individu à partir des données brutes générées par la plateforme, et
- un dispositif de quantification de l'équilibre selon l'invention, apte à communiquer avec l'unité de traitement.

**Selon d'autres caractéristiques optionnelles du système :**

- la plateforme comporte quatre capteurs de pression et/ou de force.
- la plateforme est configurée pour mesurer les valeurs de ses différents capteurs de pression et/ou de force à une fréquence supérieure ou égale à 25 Hz et de façon sensiblement constante.
- la plateforme comporte une mousse apte à déformer ou perturber des informations proprioceptives et tactiles.

L'invention porte en outre sur produit programme d'ordinateur configuré pour mettre en oeuvre le procédé de quantification selon l'invention, ledit programme d'ordinateur comportant au moins :

- un algorithme adapté pour segmenter un statokinésigramme d'un individu de façon à générer plusieurs portions de statokinésigramme,
- un algorithme adapté pour extraire, à partir des portions de statokinésigramme des valeurs d'au moins un paramètre de trajectoire,
- un algorithme adapté pour déterminer plusieurs quantificateurs, à partir des valeurs des paramètres de trajectoire extraites, et
- un algorithme adapté pour déterminer une valeur représentative de l'équilibre de l'individu à partir des valeurs desdits quantificateurs de chacune des portions de statokinésigramme.

D'autres avantages et caractéristiques de l'invention apparaitront à la lecture de la description suivante donnée à titre d'exemple illustratif et non limitatif, en référence aux Figures annexées qui représentent :

- la Figure 1, un schéma du procédé de quantification de l'équilibre selon l'invention. Les étapes encadrées en pointillée sont facultatives.

- les Figures 2, plusieurs illustrations de la mise en oeuvre du procédé de quantification de l'équilibre selon l'invention sur un statokinésigramme selon l'invention où l'axe y correspond à l'axe antéro-postérieur et l'axe x à l'axe medio-latéral, pour la figure 2B, incluant une échelle temporelle en abscisses, l'axe y1 correspond à l'axe antéro-postérieur et l'axe y2 à l'axe medio-latéral et pour la figure 2F une représentation graphique des portions du statokinésigramme en fonction du score de chaque portion.

- la Figure 3, une illustration d'une représentation graphique dans le temps des portions du statokinésigramme en fonction du score de chaque portion

- la Figure 4, un diagramme schématique d'une mise en oeuvre du procédé selon l'invention.

- la Figure 5, un dispositif de quantification de l'équilibre selon l'invention.

- la Figure 6, un système de quantification de l'équilibre selon l'invention.

## [Description de l'invention]

Dans la suite de la description, l'« équilibre » au sens de l'invention correspond à l'équilibre postural lié à la stabilité du corps et plus particulièrement à la stabilité du centre de gravité d'un individu. La notion d'équilibre selon l'invention est liée à la capacité d'un individu à éviter de chuter et englobe l'équilibre statique et l'équilibre dynamique.
La « quantification de l'équilibre » correspond, au sens de l'invention, à l'attribution d'une valeur par exemple un score, un classement ou une note à une trajectoire ou à un déplacement du centre de pression d'un individu. Cette quantification de l'équilibre permet d'obtenir une valeur représentative de l'équilibre et peut être réalisée sur la base de nombreuses échelles de tailles différentes (e.g. 1, 5, 10, 100) linéaires ou non. La valeur représentative de l'équilibre attribuée lors de la quantification de l'équilibre, peut également permettre d'affecter un individu à un groupe par exemple via une règle de décision. La quantification selon l'invention peut être réalisée notamment par la mise en oeuvre d'un algorithme de notation généré à partir d'une méthode statistique d'apprentissage ou de partitionnement.
Par « modèle » ou « règle » ou « algorithme de notation » il faut comprendre au sens de l'invention une suite finie d'opérations ou d'instructions permettant de quantifier l'équilibre, c'est-à-dire classer un ou plusieurs individus au sein de groupes préalablement définis Y, d'attribuer un score ou de hiérarchiser un ou plusieurs individus au sein d'un classement. La mise en oeuvre de cette suite finie d'opérations permet par exemple d'attribuer une étiquette $Y_0$ à une observation décrite par un ensemble de caractéristiques $X_0$ grâce par exemple à la mise en oeuvre d'une fonction f susceptible, de reproduire Y ayant observé X.

$$Y = f(X) + e$$

où e symbolise le bruit ou erreur de mesure
Par « méthode de d'apprentissage supervisé », on entend au sens de l'invention un procédé permettant de définir une fonction f à partir d'une base de n observations étiquetées ($X_{1...n}$, $Y_{1...n}$) où Y = f(X) + e. Par « méthode de partitionnement non supervisée », une méthode d'apprentissage non supervisée visant à diviser un ensemble de données en différents groupes homogènes, les groupes homogènes partageant des caractéristiques communes.
Au sens de l'invention, on entend par « centre de gravité », le centre de gravité corporel d'un individu. Il correspond au sens de l'invention au barycentre des masses de l'individu. Le centre de gravité ne peut être maintenu dans une stabilité parfaite et par exemple lors de la station debout le centre de gravité oscille d'avant en arrière et de gauche à droite.
Au sens de l'invention, on entend par « centre de pression », la projection sur le plan horizontal passant par le point de contact entre le sujet et le sol du barycentre des forces verticales exercées sur le sol par le corps du sujet (Benda, B.J. et al 1994. Biomechanical relationship between center of gravity and center of pressure during standing. Rehabilitation Engineering, IEEE Transactions on 1994, 2, 3-10). Ces mesures peuvent être réalisées grâce à une plateforme analysant la répartition des pressions sous la semelle plantaire telle une plateforme de force ou une chaussure ou un sol déformable. Sans être une projection exacte du centre de gravité, le centre de pression est fortement lié au centre de gravité. Le déplacement du centre de pression est généralement plus rapide et plus ample que celui du centre de gravité afin de le maintenir en équilibre. Il reflète les efforts effectués par un individu pour contrôler la position de son centre de gravité.
On entend par « statokinésigramme » ou « trajectoire du centre de pression », les données relatives à la trajectoire ou au déplacement du centre de pression. Le statokinésigramme peut également être appelé stabilogramme et est généralement généré par l'intermédiaire d'une plateforme telle qu'une plateforme de force, un sol « intelligent » équipé de capteurs ou des semelles équipées de capteurs de pression. Il correspond à la trajectoire calculée du centre de pression dans le temps. La trajectoire du centre de pression est définie par un ensemble de données de position dans un repère orthonormé x,y en fonction du temps et au cours d'une durée définie.

On entend par « segmenter un statokinésigramme », le fait de segmenter, échantillonner ou partitionner en fonction du temps, l'ensemble des valeurs de déplacement du centre de pression formant un statokinésigramme en plusieurs ensemble de valeurs, appelées portions du statokinésigramme.

La « plateforme » au sens de l'invention correspond à un dispositif en appui sur le sol comportant des capteurs, par exemple de type capteurs de force ou de pression, produisant un signal électrique, optique ou magnétique proportionnel à la force appliquée sur ladite plateforme par les pieds d'un individu. Les capteurs utilisés peuvent par exemple être des jauges de contrainte montées en pont de Wheastone afin de générer les 3 composantes de force et de moment Fx, Fy, Fz, Mx, My et Mz ; des capteurs de pression piézoélectriques, des capteurs de pression piézorésistifs ou des capteurs de pression capacitifs. Au sens de l'invention, la plateforme est configurée pour générer des « données brutes » issues desdits capteurs.

Par « paramètre » ou « paramètre de trajectoire » et plus particulièrement par « paramètre calculé à partir de la trajectoire du centre de pression », on entend au sens de l'invention une transformation de la trajectoire du centre de pression en un ensemble de valeurs.

Par « quantificateur » et plus particulièrement par « quantificateur calculé à partir de transformation d'un paramètre de trajectoire », on entend au sens de l'invention une seule valeur obtenue par sélection ou transformation de l'ensemble des valeurs d'un paramètre de trajectoire.

Par « quantificateur de référence », on entend une valeur obtenue à partir d'un statokinésigramme de référence provenant d'une personne dont l'équilibre a été préalablement qualifié.

Au sens de l'invention, la « courbe de ROC (Receiver Operating Characteristic) » représente l'évolution de la sensibilité (taux de vrais positifs) en fonction de la spécificité (taux de faux positifs) d'un modèle pour chaque valeur seuil donnée. C'est une courbe croissante entre le point (0,0) et le point (1,1) et en principe située au-dessus de la première bissectrice. En effet, une prédiction aléatoire donnerait une droite correspondant à la première bissectrice. Pour une courbe de ROC, plus la courbe est au-dessus de la première bissectrice, meilleure est la prédiction et l'aire sous la courbe ROC (AUC - Area Under the Curve en terminologie Anglo-Saxonne) donne un indicateur de la qualité du modèle (1 pour une prédiction idéale, 0,5 pour une prédiction aléatoire).

On entend par « traiter », « calculer », « déterminer », « afficher », « extraire » « comparer » ou plus largement « opération exécutable », au sens de l'invention, une action effectuée par un dispositif ou un processeur sauf si le contexte indique autrement. À cet égard, les opérations se rapportent à des actions et / ou des processus d'un système de traitement de données, par exemple un système informatique ou un dispositif informatique électronique, qui manipule et transforme les données représentées en tant que quantités physiques (électroniques) dans les mémoires du système informatique ou d'autres dispositifs de stockage, de transmission ou d'affichage de l'information. Ces opérations peuvent se baser sur des applications ou des logiciels.

Les termes ou expressions « application », « logiciel », « code de programme », et « code exécutable » signifient toute expression, code ou notation, d'un ensemble d'instructions destinées à provoquer un traitement de données pour effectuer une fonction particulière directement ou indirectement (e.g. après une opération de conversion vers un autre code). Les exemples de code de programme peuvent inclure, sans s'y limiter, un sous-programme, une fonction, une application exécutable, un code source, un code objet, une bibliothèque et/ou tout autre séquence d'instructions conçues pour l'exécution sur un système informatique.

On entend par « processeur », au sens de l'invention, au moins un circuit matériel configuré pour exécuter des opérations selon des instructions contenues dans un code. Le circuit matériel peut être un circuit intégré. Des exemples d'un processeur comprennent, sans s'y limiter, une unité de traitement central, un processeur graphique, un circuit intégré spécifique à l'application (ASIC) et un circuit logique programmable.

On entend par « couplé », au sens de l'invention, connecté, directement ou indirectement avec un ou plusieurs éléments intermédiaires. Deux éléments peuvent être couplés mécaniquement, électriquement ou liés par un canal de communication.

Dans la suite de la description, les mêmes références sont utilisées pour désigner les mêmes éléments.

Selon un **premier aspect,** l'invention porte sur un **procédé de quantification de l'équilibre** d'un individu pour obtenir une valeur représentative de l'équilibre dudit individu.

Le procédé de quantification 1 de l'équilibre selon l'invention repose sur le traitement des données de trajectoire du centre de pression d'un individu telles que retranscrites dans un statokinésigramme 110. Ces données de trajectoire du centre de pression d'un individu sont généralement acquises lorsque l'individu est debout.

L'influence des afférences visuelles sur la stabilité peut être mesurée en générant deux cinétiques de déplacement ou de trajectoire du centre de pression, une première les yeux ouverts et une seconde les yeux fermés. Ainsi, de préférence, l'étape de segmentation 20 est réalisée à partir d'un statokinésigramme 110 obtenu alors que l'individu a les yeux ouverts et d'un statokinésigramme 110 obtenu alors que l'individu a les yeux fermés. De façon particulière, la durée d'acquisition d'un statokinésigramme 110 peut être comprise entre 5 et 70 secondes, de préférence entre 20 et 60 secondes et de façon encore plus préférée entre 20 et 40 secondes.

De façon avantageuse, les données de trajectoire du centre de pression peuvent être obtenues lors d'un test de Romberg. Le test de Romberg consiste à placer l'individu debout, immobile, les bras le long du corps, regardant droit devant lui. Le déplacement du centre de pression est enregistré pendant 25 secondes. Par exemple, un enregistrement de 25 secondes est réalisé les yeux ouverts et un autre enregistrement de 25 secondes est réalisé les yeux fermés.

Le procédé selon l'invention peut être mis en oeuvre à partir des données relatives au déplacement du centre de pression, c'est-à-dire sur la base d'au moins un statokinésigramme 110 ou bien sur la base des données brutes issues des capteurs et relatives au déplacement du centre de pression.

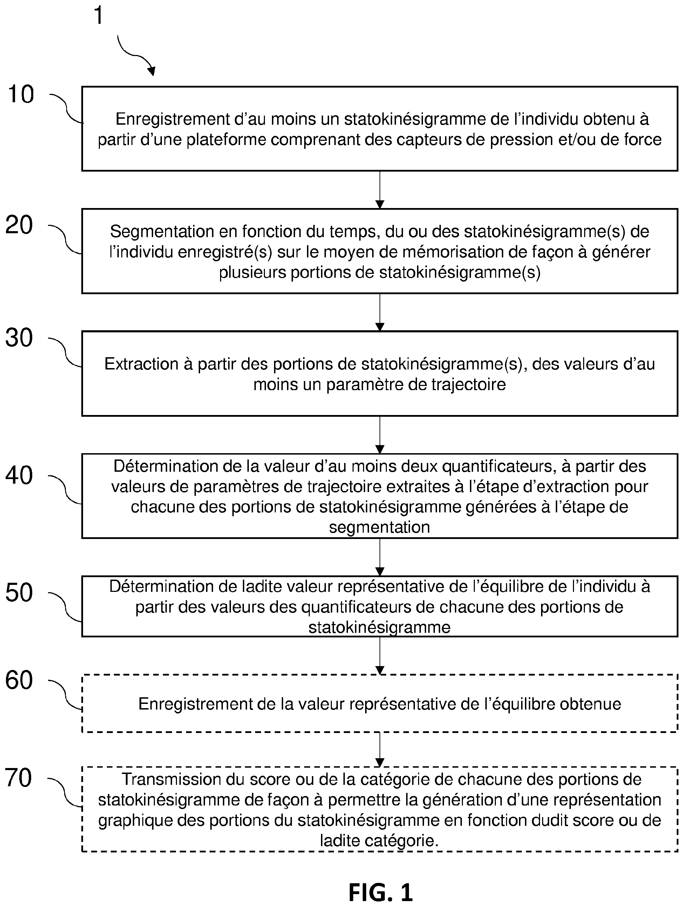

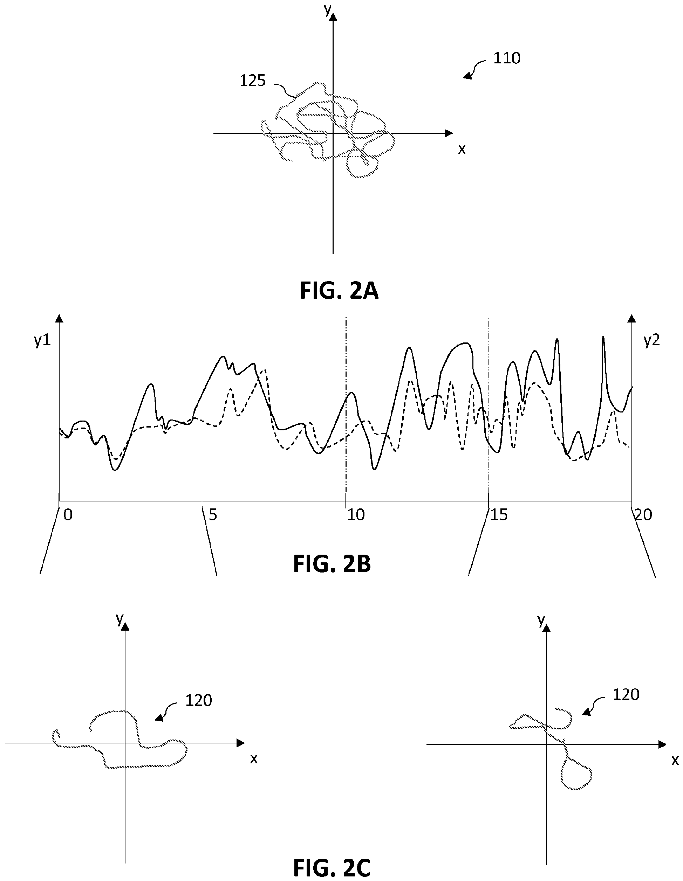

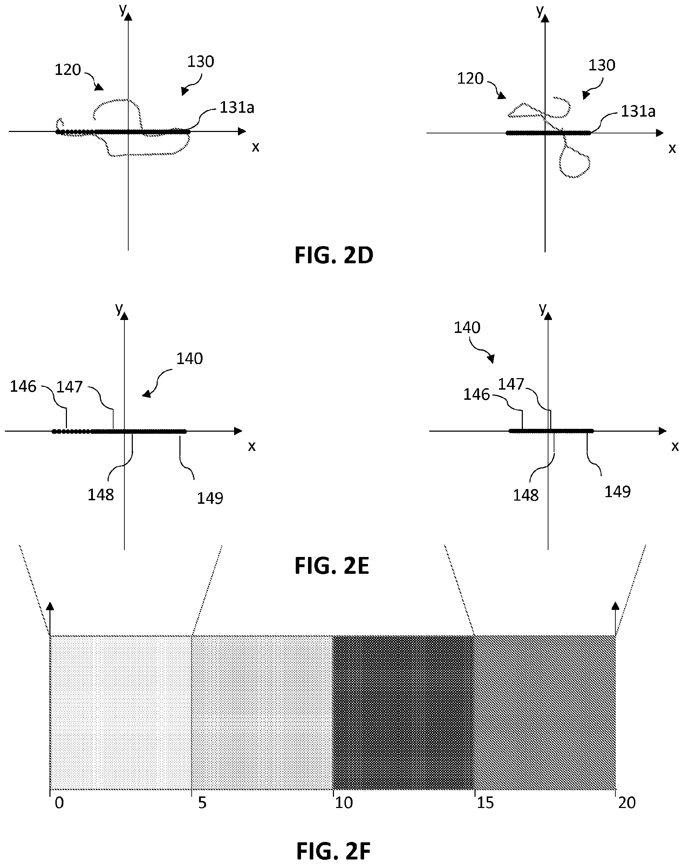

Comme présenté à la **figure 1**, le procédé selon l'invention comporte plusieurs étapes. En outre, certaines de ces étapes sont illustrées dans les **figures 2**. Le procédé selon l'invention est de préférence mis en oeuvre par un dispositif comprenant au moins un module de traitement de données 220 connecté, ou couplé, à un moyen de mémorisation 280.

Le procédé comprend notamment une **étape d'enregistrement 10** d'au moins un statokinésigramme de l'individu 110 obtenu par exemple à partir d'une plateforme 310 comprenant des capteurs 312 de pression et/ou de force. Cette étape d'enregistrement est de préférence mise en oeuvre sur le moyen de mémorisation 280. Cet enregistrement peut être réalisé sur tous types de mémoire tels que des mémoires transitoires ou non transitoires. Cet enregistrement est de préférence réalisé sur une mémoire non transitoire. Ainsi, le statokinésigramme 110 peut être généré bien avant la mise en oeuvre du procédé selon l'invention et sur un lieu distant. Alternativement, le statokinésigramme 110 peut être généré juste avant la mise en oeuvre du procédé de quantification 1 de l'équilibre selon l'invention et par un même système. Le procédé de quantification 1 selon l'invention peut donc inclure au préalable une **étape de génération des données brutes correspondant au déplacement du centre de pression 11.** Cette étape est néanmoins facultative et elle peut être réalisée en amont du procédé de quantification 1 selon l'invention par des dispositifs et des procédés connus. Les données brutes correspondant au déplacement du centre de pression sont par exemple les valeurs de pression mesurées par chacun des capteurs présents sur la plateforme. Ces données brutes peuvent faire l'objet d'une **étape de transformation 12 en données de trajectoire du centre de pression (i.e. un statokinésigramme).** Cette étape de transformation est également facultative car elle peut être réalisée en amont du procédé selon l'invention par des méthodes connues. En outre, le procédé peut comprendre une étape de ré-échantillonnage du statokinésigramme à une fréquence d'au moins 25 Hz. Ce ré-échantillonnage peut par exemple être réalisé avant l'étape de segmentation. La **figure 2A** est une représentation graphique d'un statokinésigramme 110 selon l'invention et plus particulièrement du tracé 125 du statokinésigramme 110.

Le procédé selon l'invention comporte également une **étape de segmentation 20** en fonction du temps, du ou des statokinésigramme(s) de l'individu 110 enregistré(s) sur le moyen de mémorisation 280, de façon à générer plusieurs portions 120 de statokinésigramme. Cette étape de segmentation 20 est de préférence mise en oeuvre par le module de traitement de données 220.

Les portions 120 de statokinésigramme peuvent présenter plusieurs durées. De préférence, toutes les portions d'un même statokinésigramme présentent la même durée. Cette durée peut par exemple être de cinq secondes ou moins et de façon préférée elle est inférieure ou égale à trois secondes, par exemple deux secondes ou de façon plus préférée une seconde.

Avantageusement, les portions 120 de statokinésigramme générées lors de l'étape de segmentation 20 présentent, pour des portions consécutives, un taux de recouvrement d'au moins 25 %, de préférence d'au moins 50 %. Par taux de recouvrement, il faut comprendre qu'une partie des informations contenues dans une portion n est identique à une partie des informations contenues dans une portion n+1 et qu'une autre partie des informations contenues dans une portion n est identique à une partie des informations contenues dans une portion n-1. Ainsi, dans le cas de portions d'une durée de deux secondes avec un taux de recouvrement de 25% alors les informations contenues entre t = 1,5 secondes et t = 2 secondes d'une portion n seront identiques aux informations contenues entre t = 0 secondes et t = 0,5 secondes d'une portion n+1. Avantageusement, le taux de recouvrement entre deux portions consécutives de statokinésigramme est au plus de 95 %, de façon préférée au plus de 90 %, de façon plus préférée au plus de 80 % et de manière encore plus préférée au plus de 75 %.

De façon préférée, l'étape de segmentation 20 permet la génération, pour chaque statokinésigramme, d'au moins dix portions 120 de statokinésigramme, de façon plus préférée au moins vingt et de façon encore plus préférée au moins trente portions 120 de statokinésigramme. La **figure 2B** illustre par exemple les données du statokinésigramme de la figure 2A représentées sur un axe temporel de 20 secondes, ledit statokinésigramme étant ici segmenté en quatre portions de chacune cinq secondes. Les données de déplacement associées aux portions 0-5 secondes et 15-20 secondes sont représentées sur la **figure 2C.**

Le procédé selon l'invention comporte également une **étape d'extraction 30** à partir des portions 120 de statokinésigramme, des valeurs d'au moins un paramètre de trajectoire 130. Cette étape d'extraction 30 est de préférence mise en oeuvre par le module de traitement de données 220.

Le paramètre de trajectoire 130 est de préférence un paramètre de trajectoire de position, de stabilité et/ou de dynamique, du centre de pression. Ainsi, l'étape d'extraction 30, par le module de traitement de données 220 et à partir des portions 120 de statokinésigramme de l'individu comprend l'extraction 30 des valeurs d'au moins un paramètre 130 de trajectoire :

- de position 131 du centre de pression,
- de stabilité 132 du centre de pression, et/ou
- de dynamique 133 du centre de pression.

En outre, l'analyse de plusieurs paramètres provenant de différentes familles de paramètres est particulièrement avantageuse. Ainsi, l'étape d'extraction 30, par le module de traitement de données 220 et à partir de portions 120 du statokinésigramme de l'individu comprend l'extraction 30 des valeurs d'au moins deux paramètres de trajectoire :

- de position 131 du centre de pression,
- de stabilité 132 du centre de pression, et/ou
- de dynamique 133 du centre de pression.

De façon plus particulière, le **paramètre de trajectoire de position 131** du centre de pression peut être sélectionné parmi :

- La position du centre de pression selon l'axe X : cette position correspond à la position du centre de pression par rapport à la ligne médiane du repère orthonormé dans un plan de l'axe X. Par exemple, en cas de position décalée du centre de pression vers la gauche à un instant t, la position selon l'axe X présente, pour cet instant t une valeur négative signe d'un hyper appui gauche. Cette position peut par exemple être mesurée en millimètre. Par exemple, la **figure 2D** représente la projection sur l'axe X de l'ensemble des valeurs du paramètre « position du centre de pression selon l'axe X » 131a, obtenues à partir d'une portion 120 du statokinésigramme 110 présenté en figure 2A ;
- La position du centre de pression selon l'axe Y : cette position correspond à la position du centre de pression par rapport à la ligne médiane du repère orthonormé dans un plan de l'axe Y. Par exemple, en cas de position décalée vers l'arrière à un instant t, la position selon l'axe Y présente, pour cet instant t une valeur négative signe d'un hyper appui postérieur. Cette position peut par exemple être mesurée en millimètre.
- Le rayon en coordonnées polaires : cette distance correspond à l'éloignement du centre de pression par rapport à la position moyenne du centre de pression selon le plan orthonormé (0,0). Par exemple, en cas de décalage de 4 millimètres du centre de pression par rapport à la position moyenne du centre de pression selon un axe de 60°, à un instant t, le rayon en coordonnées polaires présente, pour cet instant t, une valeur de 4 millimètres. Une telle transformation, proposée par les inventeurs permet de quantifier l'éloignement global du centre de pression par rapport à un point d'origine sans se limiter aux coordonnées X et Y du centre de gravité. Le rayon en coordonnées polaires est calculé pour l'ensemble des points d'échantillonnage du déplacement du centre de pression sur la durée de l'acquisition.

De façon plus particulière, le **paramètre de trajectoire de stabilité 132** du centre de pression peut être sélectionné parmi :

- L'équilibre radial : il correspond à l'éloignement maximal du centre de pression de sa valeur courante à un instant donné sur une durée prédéfinie de t secondes. La durée prise en compte pour le calcul de l'équilibre radial peut être comprise entre 0,05 à 10 secondes, de préférence de 0,1 à 2 secondes. L'équilibre radial est calculé pour l'ensemble des points d'échantillonnage du déplacement du centre de pression sur la durée de l'acquisition. Il est ainsi fonction du temps et peut être mesuré par exemple en millimètres.
- L'équilibre temporel : il correspond au temps nécessaire pour que le centre de pression s'éloigne de plus de r millimètres de sa position courante à un instant donné. La distance r prise en compte pour le calcul de l'équilibre temporel peut être comprise entre 0,1 à 20 millimètres, de préférence de 1 à 10 millimètres. L'équilibre temporel est calculé pour l'ensemble des points d'échantillonnage du déplacement du centre de pression sur la durée de l'acquisition. Il est ainsi fonction du temps et peut être mesuré par exemple en secondes.
- L'intervalle balistique : cela correspond à l'intervalle de temps entre deux positions d'équilibre. De préférence, une position d'équilibre correspond à un moment où l'équilibre temporel est supérieur à 60%, de préférence supérieur à 80 %, du maximum observé sur le statokinésigramme. L'intervalle balistique est calculé sur la durée de l'acquisition et le nombre de valeurs obtenues peut varier en fonction des acquisitions. Il n'est pas fonction du temps et peut être mesuré par exemple en secondes.

De façon plus particulière, le **paramètre de trajectoire de dynamique 133** du centre de pression peut être sélectionné parmi :

- La vitesse du déplacement du centre de pression : elle est calculée pour l'ensemble des points d'échantillonnage du déplacement du centre de pression sur la durée de l'acquisition. Ce paramètre est ainsi fonction du temps et peut être mesuré par exemple en millimètres par secondes.
- L'accélération du déplacement du centre de pression : elle est calculée pour l'ensemble des points d'échantillonnage du déplacement du centre de pression sur la durée de l'acquisition. Il est ainsi fonction du temps et peut être mesuré par exemple en millimètres par secondes au carré.
- La puissance : elle correspond à la valeur du produit scalaire de la vitesse et de l'accélération calculée pour l'ensemble des points d'échantillonnage. Ce paramètre représente l'énergie dépensée par l'individu pour modifier la norme de la vitesse du centre de pression.
- La déviation : elle correspond à la norme du produit vectoriel de la vitesse et de l'accélération calculée pour l'ensemble des points d'échantillonnage. Ce paramètre représente l'énergie dépensée par l'individu pour modifier la direction de la vitesse du centre de pression (i.e son déplacement, sa trajectoire ou son mouvement). Dans le cadre de l'étude d'une portion 120 de statokinésigramme, la puissance et la déviation sont deux paramètres pouvant avantageusement remplacer la longueur du déplacement du centre de pression, la variance de la vitesse en fonction de l'axe Y (VFY) ou encore la LFS (i.e. rapport de la longueur sur la surface), renseignant selon l'art antérieur sur la consommation d'énergie dépensée par le sujet pour contrôler sa posture. En particulier la déviation, proposée par les inventeurs, permet d'évaluer sous un nouvel angle la dépense énergétique de l'individu et est plus pertinente dans le procédé selon l'invention que les paramètres proposés dans l'art antérieur (e.g. VFY, LFS).

Le procédé selon l'invention comporte également une **étape de détermination 40** de la valeur d'au moins deux quantificateurs 140, à partir des valeurs de paramètres de trajectoire 130 extraites à l'étape d'extraction 30 pour chacune des portions 120 de statokinésigramme générées à l'étape de segmentation 20. Cette étape de détermination 40 de la valeur d'au moins deux quantificateurs 140 est de préférence mise en oeuvre par le module de traitement de données 220.

Dans le procédé développé par les inventeurs, ils se sont intéressés de façon individuelle à chacune des portions de statokinésigramme générées lors de l'étape de segmentation. Ainsi, à partir des valeurs d'un ou plusieurs paramètres de trajectoire, ils ont calculé pour chacune des portions au moins deux quantificateurs. Avantageusement, le procédé comprend, à l'étape de détermination 40, la détermination de la valeur d'au moins trois quantificateurs 140, de façon préférée d'au moins cinq quantificateurs. Cela conduit à la génération lors de l'étape de détermination 40 d'une pluralité de quantificateurs associés à des portions 120 de statokinésigramme.

Il existe une pluralité de quantificateurs 140 possible. Dans ce contexte, les inventeurs se sont intéressés à de nombreux procédés de transformations des paramètres de trajectoire 130 afin de générer, à partir de ces ensembles de valeurs et si nécessaire en combinaison avec d'autres ensembles de valeurs, une seule valeur représentative de l'équilibre d'un individu. Ainsi, de nouveaux quantificateurs 140 jamais divulgués par le passé ont été générés.

L'étape de détermination 40 de plusieurs quantificateurs 140 à partir des paramètres de trajectoire 130 consiste plus particulièrement à transformer, pour chaque paramètre de trajectoire 130, l'ensemble des valeurs en une seule valeur pouvant être utilisée dans le cadre d'une comparaison par exemple par l'intermédiaire d'un modèle statistique. Cette transformation en une seule valeur (quantificateur) peut être répétée pour plusieurs paramètres de trajectoire 130 ou pour un même paramètre de trajectoire. Par exemple, **la figure 2E** représente la projection sur l'axe X de l'ensemble des valeurs du paramètre « position du centre de pression selon l'axe X » ainsi que, pour chaque graphique, quatre quantificateurs calculés à partir des valeurs de ce paramètre.

Les quantificateurs 140 les plus informatifs dans le cadre du procédé de quantification selon l'invention sont la valeur moyenne, la variance, de la moyenne des carrés, ou une valeur extrême, d'un paramètre de trajectoire 130 extrait. Ainsi, de façon préférée, le procédé de quantification 1 selon l'invention comporte, pour au moins un paramètre, le calcul de la valeur moyenne 147, de la valeur médiane 148, de la variance, de la moyenne des carrés ou d'au moins une valeur extrême 146, 149, dudit paramètre de trajectoire 131, 132, 133.

Les valeurs extrêmes d'un paramètre sont obtenues via la détermination d'un percentile. Un percentile, ou centile, peut par exemple être calculé en ordonnant puis triant l'ensemble des valeurs d'un paramètre en 100 sous-ensembles comprenant un même nombre de valeurs. La figure 2E représente le percentile 10 146 et le percentile 95 149 du paramètre « position du centre de pression selon l'axe X » correspondant respectivement à la valeur la plus élevée de la position du centre de pression selon l'axe X au sein des 10 % de valeurs les plus faibles et la valeur la plus faible de la position du centre de pression selon l'axe X au sein des 5 % de valeurs les plus élevée. Ainsi, le percentile 10 est la valeur séparant 10 % des valeurs les plus faibles et 90 % des valeurs les plus élevées alors que le percentile 95 est la valeur séparant 95 % des valeurs les plus faibles et 5 % des valeurs les plus élevées De préférence, une valeur extrême d'un paramètre de trajectoire 130 correspond à un percentile inférieur ou égal à 15 ou bien à un percentile supérieur ou égal à 85 ; de façon plus préférée, à un percentile inférieur ou égal à 10 ou bien à un percentile supérieur ou égal à 90 et de façon encore plus préférée à un percentile inférieur ou égal à 5 ou bien à un percentile supérieur ou égal à 95. Alternativement, une valeur extrême d'un paramètre de trajectoire 130 peut correspondre à un percentile supérieur ou égal au percentile 5 et inférieur ou égal au percentile 15 (valeurs extrêmes faibles) ou à un percentile supérieur ou égal au percentile 85 et un percentile inférieur ou égal au percentile 95 (valeurs extrêmes élevées).

En outre, au-delà du calcul d'un quantificateur 140, les inventeurs ont montré que la comparaison yeux ouverts (O) / yeux fermés (F) sur certains paramètres ou quantificateurs permettrait de différencier des performances en termes d'équilibre. Ainsi, de façon particulière, le procédé de quantification 1 selon l'invention comporte pour au moins un quantificateur, le calcul d'un rapport O/F ou F/O.

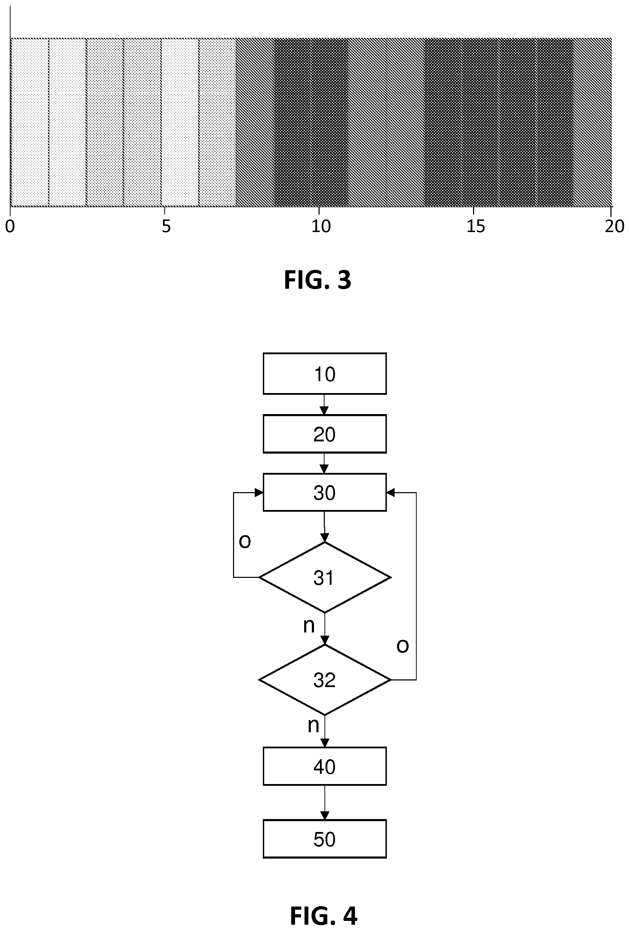

La **figure 4** présente un mode de réalisation des premières étapes du procédé selon l'invention. Le procédé illustré débute par une étape d'enregistrement 10 d'un statokinésigramme 110 d'un individu. Le statokinésigramme 110 est ensuite segmenté, par exemple en portions d'une durée de 1 seconde avec un taux de recouvrement de 50%.

Le procédé comporte ensuite une étape d'extraction 30 des valeurs d'un paramètre de trajectoire 130 d'une portion de statokinésigramme. Ensuite, le procédé détermine 31 s'il doit extraire des valeurs d'un autre paramètre de trajectoire 130. Si oui (o sur la figure) alors l'étape d'extraction 30 des valeurs d'un paramètre de trajectoire 130 est répétée sur la même portion de statokinésigramme. Si non (n sur la figure), le procédé détermine 32 s'il reste une portion 120 de statokinésigramme n'ayant pas été traitée. Si oui (o sur la figure) alors l'étape d'extraction 30 des valeurs d'un paramètre de trajectoire 130 est répétée sur une nouvelle portion 120 de statokinésigramme. Si non (n sur la figure), le procédé procède à l'étape de détermination 40 de la valeur d'au moins deux quantificateurs 140 à partir des valeurs de paramètre de trajectoire. Le procédé réalise ces calculs jusqu'à que tous les quantificateurs nécessaires aient été calculés. Ensuite, il initie la phase de détermination 50 de la valeur représentative de l'équilibre dudit individu.

Ainsi, le procédé selon l'invention comporte également une **étape de détermination 50** de ladite valeur représentative de l'équilibre de l'individu à partir des valeurs des quantificateurs de chacune des portions 120 de statokinésigramme. Cette étape de détermination 50 est de préférence mise en oeuvre par le module de traitement de données 220. De façon préférée, l'étape de détermination 50 peut être précédée d'une étape de normalisation des quantificateurs 140.

Avantageusement, l'étape de détermination 50 est réalisée en mettant en oeuvre les valeurs des quantificateurs 140 déterminés à l'étape de détermination 40 dans un algorithme de notation 500, de préférence préalablement calibré. L'algorithme de notation 500 peut être préalablement calibré sur la base des valeurs des quantificateurs 140 ou bien sur la base des valeurs des mêmes quantificateurs obtenus à partir de portions de statokinésigrammes de référence 121.

De façon préférée, la mise en oeuvre de l'algorithme de notation 500 peut comprendre l'attribution d'un score à chacune des portions de statokinésigrammes 120 en fonction des valeurs des quantificateurs. Le score peut par exemple être un score représentatif d'une caractéristique du déplacement du centre de pression (régulier ou irrégulier) ou encore être représentatif de la qualité de l'équilibre de l'individu sur chaque portion du statokinésigramme. Par exemple, dans la **figure 2F et la figure 3,** le procédé selon l'invention permet de donner un score à chacune des portions du statokinésigramme qui est représenté par une coloration en nuance de gris plus ou moins sombre.

Alternativement, la mise en oeuvre de l'algorithme de notation 500 peut comprendre le classement de chacune des portions 120 de statokinésigrammes en catégories. Par exemple, le procédé peut permettre d'associer chaque portion à une catégorie telle que « équilibre bon » ou « équilibre à risque ». La catégorie peut être choisie parmi au moins deux catégories, par exemple parmi cinq catégories et est de préférence choisie parmi deux catégories.

Lors de l'attribution d'un score ou d'un classement dans une catégorie d'une portion 120 de statokinésigramme, le procédé selon l'invention peut comprendre la prise en compte d'une pluralité de valeurs de quantificateurs 140.

Comme cela sera présenté dans les exemples, la classification peut être non supervisée et permettre la discrimination entre une famille de portions ayant été la plus fréquente appelée périodes régulières - RP, tandis que la moins commune, qui se caractérisera par une dynamique moins stable, sera considérée comme des périodes irrégulières - IP. Ainsi, il peut être considéré que la seconde famille sera plus importante et plus fréquente dans les statokinésigrammes des personnes plus propices à chuter que dans les statokinésigrammes des personnes ayant un bon équilibre. Par conséquent, cette différence de proportion peut être utilisée pour déterminer une valeur représentative de l'équilibre chez les individus.

Cet algorithme de notation 500 peut avoir été construit à partir de différents modèles d'apprentissage, notamment de partitionnement, supervisés ou non supervisés.

L'algorithme de notation 500 est de façon préférée un algorithme de partitionnement non supervisé. Cet algorithme de partitionnement non supervisé peut par exemple être sélectionné parmi un modèle de mélange Gaussien non supervisé, une classification ascendante hiérarchique (Hierarchical clustering Agglomerative en terminologie Anglo-Saxonne), une classification descendante hiérarchique (Hierarchical clustering divisive en terminologie Anglo-Saxonne). De préférence, c'est un modèle de mélange Gaussien non supervisé.

Alternativement, cet algorithme de notation 500 repose sur un modèle d'apprentissage statistique supervisé configuré de façon à minimiser un risque de la règle d'ordonnancement et ainsi permettant d'obtenir des règles de prédiction plus performantes. Dans ce cas, la détermination 50 peut comprendre une étape de comparaison par le module de traitement de données 220 desdites valeurs d'au moins deux quantificateurs 140 à des valeurs prédéterminées. Les valeurs prédéterminées peuvent par exemple être calculées à partir de portions de statokinésigrammes de référence 121 enregistrés dans une base de données. L'étape de comparaison peut être basée sur un modèle, entrainé sur un jeu de données et configuré pour prédire l'étiquette d'une portion statokinésigramme ou d'un statokinésigramme complet. Par exemple, aux fins de la calibration, il est possible d'utiliser un jeu de données provenant d'un ensemble d'individus représentatifs d'une population, caractérisés par plusieurs portions de statokinésigrammes de références 121 et leurs paramètres et/ou quantificateurs de référence associés et par une étiquette (label ou classe) binaire, par exemple de la forme « bon équilibre » / « mauvais équilibre ». Le jeu de donnée peut également comprendre des étiquettes multiples. Dans le cadre de la présente invention le procédé de quantification 1 peut reposer sur au moins vingt-cinq portions de statokinésigrammes de référence 121, de préférence au moins cinquante, et de façon encore plus préférée au moins cent. L'étape de comparaison peut alors comporter l'utilisation d'un modèle d'apprentissage statistique supervisé sélectionné par exemple parmi les méthodes à noyau (e.g. Séparateurs à Vaste Marge - Support Vector Machines SVM, Kernel Ridge Regression) décrites par exemple dans Burges, 1998 (Data Mining and Knowledge Discovery. A Tutorial on Support Vector Machines for Pattern Recognition), les méthodes d'ensembles (e.g. Bagging, Boosting, arbres de décision, Random Forest) décrites par exemple dans Brieman, 2001 (Machine Learning. Random Forests), ou les réseaux de neurones décrits par exemple dans Rosenblatt, 1958 (The perceptron: a probabilistic model for information storage and organization in the brain).

En outre, la création de l'algorithme de notation 500 peut inclure une étape de « Bagging » et/ou une étape de Boosting. Le bagging et sa mise en oeuvre sont décrits en détail dans Galar et al 2011 (A Review on Ensembles for the Class Imbalance Problem: Bagging-, Boosting-, and Hybrid-Based Approaches). Le boosting regroupe un ensemble d'algorithmes tels que : Adaboost, LPBoost, TotalBoost, BrownBoost, xgboost, MadaBoost, LogitBoost. Le Boosting est une méthode séquentielle et chaque échantillon est tiré en fonction des performances de la règle de base sur l'échantillon précédent. Le Boosting et sa mise en oeuvre sont décrits en détail dans Freund & Schapire 1999 (Machine Learning Large. Margin Classification Using the Perceptron Algorithm).

Le procédé de quantification 1 selon l'invention permet d'obtenir une quantification de l'équilibre sous la forme d'un score ou d'une valeur entre zéro et cent, proportionnels à la qualité de l'équilibre. Par exemple, une valeur inférieure à trente indique un équilibre faible

Dans ce contexte, le procédé peut comprendre la prise en compte des valeurs de quantificateurs obtenus, des classements de portions ou des scores de portion provenant de plusieurs statokinésigrammes pour la détermination de la valeur représentative de l'équilibre. Lorsque les statokinésigrammes 110 ont été générés lors d'un test de Romberg, il y a obtention d'un statokinésigramme « yeux ouverts » et d'un statokinésigramme « yeux fermés ». Les scores obtenus à partir chacune des portions de statokinésigrammes « yeux ouverts » et « yeux fermés » sont avantageusement pris en compte de façon à obtenir une valeur représentative de l'équilibre de l'individu.

De façon préférée, l'étape de détermination 50 est suivie par **une étape d'enregistrement 60 de la valeur représentative de l'équilibre obtenue** et éventuellement l'association de ladite valeur à un identifiant unique lié audit individu.

Ainsi, cela permet à l'individu de comparer sa valeur représentative de l'équilibre au cours du temps. Ainsi, de façon préférée, le procédé de quantification 1 selon l'invention peut être mis en oeuvre chez un même individu à des dates différentes de façon à suivre l'évolution de sa valeur représentative de l'équilibre et donc de la qualité de son équilibre.

En outre, avantageusement, le procédé selon l'invention peut comporter une étape de **génération 70 d'instruction** permettant une représentation graphique des scores de chacune des portions de statokinésigrammes 120. De façon préférée, au moins un graphique est généré automatiquement à la fin de l'étape de détermination 50 de ladite valeur représentative de l'équilibre. Le procédé de quantification 1 de l'équilibre selon l'invention peut également comprendre une étape de représentation graphique de ladite valeur représentative de l'équilibre. La valeur peut faire l'objet d'un affichage par l'intermédiaire d'un module d'affichage. Cet affichage peut être un affichage simple indiquant une valeur ou bien une représentation graphique. Le procédé peut également comprendre une étape de représentation graphique, sur un moyen de visualisation 270, de la valeur d'au moins un quantificateur 140, d'un score ou d'une catégorie pour chacune des portions 120 de statokinésigramme générées à l'étape de segmentation 20 en fonction du temps. La figure 3 présente un exemple de graphique pouvant être généré lors de la mise en oeuvre du procédé selon l'invention. Le graphique de la figure 3 comporte seize portions dont la couleur est fonction du score de chacune des portions. Le score peut être représentatif de la régularité ou de l'irrégularité du déplacement du centre de pression dans cette portion. L'étude de la figure 3 montre que le déplacement du centre de pression devient de plus en plus irrégulier alors que le déplacement était plutôt régulier en début d'acquisition.

Le procédé selon l'invention peut également permettre la représentation graphique de l'évolution de la valeur représentative de l'équilibre dans le temps ou bien le placement de cette valeur au sein d'un groupe d'individus.

Ladite valeur représentative de l'équilibre peut également être transmise sur des systèmes distants tels que des tablettes, des serveurs ou des ordinateurs personnels. Ainsi, le procédé de quantification 1 selon l'invention peut comprendre une étape de transmission de la valeur représentative de l'équilibre, des quantificateurs calculés et/ou des paramètres calculés vers au moins un système communicant tel qu'une tablette, un serveur ou un ordinateur, via au moins un réseau de communication.

De préférence, l'invention porte sur un procédé de quantification 1 de l'équilibre comprenant la quantification de l'équilibre statique et de l'équilibre dynamique. De façon encore plus préférée, l'invention porte sur la quantification de l'équilibre statique.

Dans le cadre du développement de ce nouveau procédé de quantification 1 de l'équilibre, les inventeurs ont vérifié la pertinence de la valeur représentative de l'équilibre obtenue via les modèles statistiques développés et notamment des algorithmes de notation utilisés par l'intermédiaire de courbes de ROC. Les modèles statistiques développés et notamment les algorithmes de notation 500 utilisés par les inventeurs permettent d'obtenir des AUC supérieurs à 0,75.

**Selon un aspect, l'invention porte sur un dispositif de quantification 2 de l'équilibre** apte à mettre en oeuvre le procédé de quantification 1 de l'équilibre selon l'invention. Plus particulièrement, le dispositif de quantification 2 de l'équilibre selon l'invention comporte :

- un module de communication 210, apte à recevoir des données comprenant au moins un statokinésigramme 110 dudit individu,
- un moyen de mémorisation 280, apte à enregistrer le statokinésigramme 110, et
- un module de traitement de données 220.

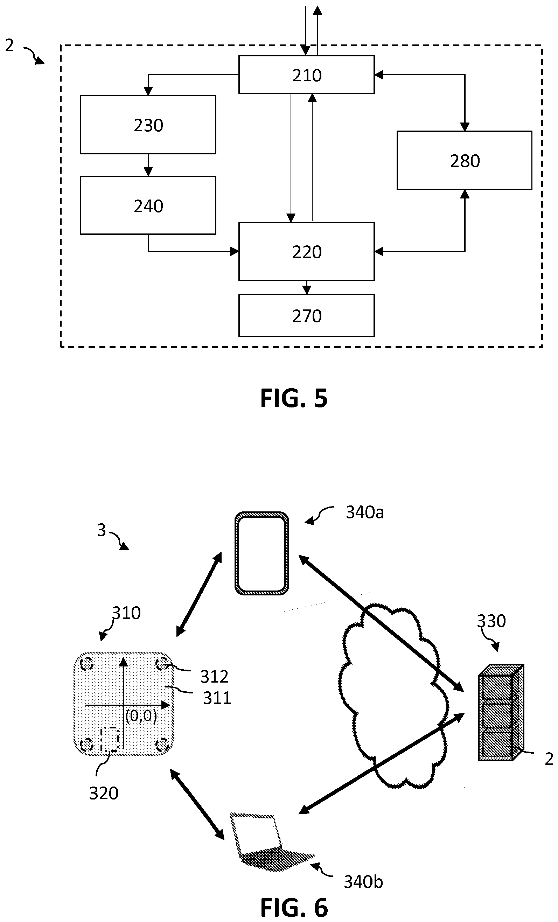

Un dispositif de quantification de l'équilibre 2 selon l'invention est représenté schématiquement à la **figure 5.**

Le **module de communication 210** est configuré pour recevoir et transmettre des informations à des systèmes distants tels que des plateformes, des tablettes, des téléphones, des ordinateurs ou des serveurs. Le module de communication permet de transmettre les données sur au moins un réseau de communication et peut comprendre une communication filaire ou sans fil. De préférence la communication est opérée par l'intermédiaire d'un protocole sans fils tel que wifi, 3G, 4G, et/ou Bluetooth.

Le module de communication 210 permet par exemple de recevoir et de transmettre des informations à des systèmes distants tels que des tablettes, des téléphones, des ordinateurs ou des serveurs. Ces informations peuvent être des données brutes de déplacement du centre de pression ou des statokinésigrammes 110. Il est également configuré pour envoyer les données relatives aux portions, aux paramètres calculés, aux quantificateurs calculés, et la valeur représentative de l'équilibre. Ces échanges de données peuvent prendre la forme d'envoi et de réception de fichiers contenants les valeurs brutes des capteurs de pression, de fichiers contenant les coordonnées de la trajectoire du centre de pression, et de fichiers comportant les portions 120, les paramètres de trajectoire 130, les quantificateurs 140, et les valeurs représentatives de l'équilibre déterminés à partir de portions 120 de statokinésigramme. Les données échangées peuvent de préférence être transférées de façon cryptée et associées à une clé spécifique de l'individu étudié. Le module de communication 210 est en outre apte à à permettre la communication entre le dispositif 2 et un terminal distant, dont un client 400. Le client est généralement, tout matériel et/ou logiciel susceptible d'accéder au dispositif de quantification selon l'invention.

Le **module de traitement de données 220** est configuré pour :

- Segmenter, en fonction du temps, le statokinésigramme 110 d'un individu, transmis par le module de communication 210, de façon à générer plusieurs portions 120 de statokinésigramme,
- Extraire, à partir des portions 120 de statokinésigramme, des valeurs d'au moins un paramètre de trajectoire 130,
- Déterminer plusieurs quantificateurs 140, à partir des valeurs des paramètres de trajectoire 130 extraites,
- Déterminer une valeur représentative de l'équilibre de l'individu à partir des valeurs desdits quantificateurs 140 de chacune des portions 120 de statokinésigrammes.

De façon préférée, le module de traitement de données 220 est configuré pour mettre en oeuvre les différentes étapes du procédé de quantification 1 selon l'invention. Ainsi, les étapes préférées du procédé de quantification 1 de l'équilibre selon l'invention sont également des configurations préférées pour le module de traitement de données 220 selon l'invention.

Le module de traitement de données 220 comporte avantageusement un processeur et est apte à se connecter à un moyen de mémorisation 280.

Le **moyen de mémorisation 280** peut comprendre une mémoire transitoire et/ou une mémoire non transitoire. Il est apte à enregistrer, par exemple sous la forme de fichiers, les valeurs brutes des capteurs de pression, les coordonnées de la trajectoire du centre de pression, les portions 120, les paramètres de trajectoire 130, les quantificateurs 140, et les valeurs représentatives de l'équilibre déterminés à partir des portions 120 de statokinésigramme(s). La mémoire non transitoire permet par exemple d'enregistrer la configuration du module de traitement de données alors que la mémoire non transitoire permet par exemple d'enregistrer le statokinésigramme 110. La mémoire non transitoire peut être un support tel qu'un CDrom, une carte mémoire, ou un disque dur hébergé par un serveur distant.

Le dispositif de quantification 2 selon l'invention peut également comporter un **module de génération 230 de statokinésigramme 110.** Ce module est configuré pour générer les données relatives à un statokinésigramme 110 (e.g. position selon les axes x et y en fonction du temps) à partir de données brutes de déplacement du centre de pression telles qu'elles peuvent être générées par des capteurs de force ou de pression.

Le dispositif de quantification 2 selon l'invention peut également comporter **un module de ré-échantillonnage 240.** En effet, les dispositifs aptes à générer des données brutes de déplacement du centre de pression ou des statokinésigramme 110 ne proposent pas tous une fréquence d'échantillonnage contrôlée. Ainsi, certains dispositifs peuvent entrainer la génération de statokinésigramme 110 présentant une première fréquence aléatoire, dont il n'est pas possible de prédire la fréquence car elle varie constamment au cours de l'acquisition, par exemple comprise, pour un même statokinésigramme 110, entre 10 et 1000 Hz. Or, une telle variation de fréquence peut entrainer des diminutions de la performance du procédé de quantification 1 de l'équilibre selon l'invention. Ainsi, de préférence, le module de ré-échantillonnage est configuré pour traiter les données brutes ou les statokinésigramme 110 à une première fréquence de façon à générer des statokinésigrammes 110 ré-échantillonnés à une seconde fréquence et présentant une fréquence sensiblement constante. Par fréquence sensiblement constante, il faut comprendre une fréquence variant de moins de 10 % au sein du statokinésigramme 110, de préférence variant de moins de 5 %, de façon encore plus préférée variant de moins de 1 %. Le statokinésigramme 110 à une deuxième fréquence, généré par le module de ré-échantillonnage 240, présente une fréquence d'échantillonnage au moins égale à 25 Hz. De préférence, la deuxième fréquence est sensiblement identique à la fréquence des statokinésigrammes de références 111.

Le dispositif de quantification de l'équilibre selon l'invention peut comprendre un **module de débruitage** configurée pour filtrer les données brutes générées par les capteurs de pression ou de force de façon à réduire ou supprimer les signaux parasites. Le débruitage peut être basé sur des diverses méthodes telles que le débruitage par ondelette, seuillages, filtre de Wiener et déconvolution.

Le dispositif peut également comprendre une **interface de contrôle.** Cette interface de contrôle est configurée pour permettre l'interaction d'un utilisateur avec le dispositif de quantification de l'équilibre. Elle peut comprendre par exemple des actionneurs manuel (e.g. boutons) ou un écran tactile aptes à recevoir les commandes de l'utilisateur.

Le dispositif peut également comprendre un **module d'affichage ou moyen de visualisation 270.** Ce module d'affichage peut comprendre un écran à cristaux liquides. Il permet d'afficher différentes informations telles que les résultats de la quantification, la valeur représentative de l'équilibre, la progression dans le temps de ladite valeur et son positionnement par rapport aux valeurs représentatives de l'équilibre au sein d'un groupe de personne.

Comme cela vient d'être présenté, le dispositif 1 d'acquisition selon l'invention comprend une pluralité de modules. Ces modules sont distincts sur la figure 5 mais l'invention peut prévoir divers types d'agencement comme par exemple un seul module cumulant l'ensemble des fonctions décrites ici. De même, ces moyens peuvent être divisés en plusieurs cartes électroniques ou bien rassemblés sur une seule carte électronique. De même, lorsque l'on prête une action à un dispositif ou un module celle-ci est en fait effectuée par un microprocesseur du dispositif ou module commandé par des codes instructions enregistrés dans une mémoire. Si l'on prête une action à une application, celle-ci est en fait effectuée par un microprocesseur du dispositif dans une mémoire duquel les codes instructions correspondant à l'application sont enregistrés. Lorsqu'un dispositif ou module émet ou reçoit un message, ce message est émis ou reçu par une interface de communication. Les mémoires évoquées dans cette invention peuvent correspondre à une mémoire vive et/ou une mémoire de masse. La mémoire de masse peut- être un support tel qu'une carte mémoire, ou un disque dur hébergé par un serveur distant.

**Selon un autre aspect, l'invention porte sur un système 3 de quantification de l'équilibre** représenté sur la **figure 6,** adapté pour mettre en oeuvre le procédé de quantification 1 selon l'invention. De préférence, le système 3 de quantification de l'équilibre d'un individu, comprend :

- une plateforme 310, ladite plateforme 310 étant adaptée à recevoir un individu et comprenant des capteurs 312 de pression et/ou de force configurés pour générer des données brutes 313, à une première fréquence, fonction d'une pression exercée par les pieds de l'individu sur la plateforme 310,
- une unité de traitement 320 des données brutes, agencée pour obtenir au moins un statokinésigramme 110 de l'individu à partir des données brutes 313 générées par la plateforme 310, et
- un dispositif de quantification de l'équilibre 2 décrit précédemment, apte à communiquer avec l'unité de traitement 320.

Comme cela est présenté à la figure 6, **la plateforme 310** selon l'invention est un support destiné à recevoir un individu et apte à mesure le déplacement d'un centre de pression grâce à des capteurs de forces et/ou de pression. Tout système de capteur permettant de mesurer le centre de pression peut être utilisé. Le seul prérequis est que la plateforme 310 soit en mesure de produire des données brutes permettant de positionner le centre de pression. Ce support peut par exemple être une paire de semelle ou au moins un plateau. De façon préférée la plateforme 310 comporte un **plateau 311.** D'une manière générale, les dimensions d'un côté du plateau 311 pourront être comprises entre 15 et 70 cm, de préférence de l'ordre de 25 à 40 cm. Ce plateau 311 peut par exemple comporter un gabarit permettant un positionnement reproductible des pieds, entre individus et au cours du temps pour un même individu.

La plateforme 310 est configurée pour mesurer la pression ou les forces appliquées sur le plateau à un instant donné et comporte pour cela des **capteurs 312.** Les capteurs vont transformer la force appliquée en un signal électrique, optique ou magnétique correspondant aux données brutes. Ces données brutes peuvent être combinées et traitées de façon à préciser les coordonnées du centre des pressions et de suivre ses variations dans le temps. Ces capteurs peuvent être des capteurs de pressions ou de force. Un capteur de force mesure la résultante des forces d'appui d'un sujet debout. La mesure des forces et moments exercés au niveau de la plateforme permet de préciser les coordonnées du centre de pression et de suivre ses variations dans le temps. Un capteur de pression peut comprendre par exemple une cellule de pression configuré pour mesurer ou détecter la pression induite par le poids de l'individu placé sur le plateau ou la pression exercé par les pieds de l'individu sur la plateforme. Les données issues de ces capteurs sont les données brutes. La plateforme 310 peut également comprendre d'une pluralité de capteurs résistifs ou piezo électrique (par exemple entre 1000 et 6000 capteurs). Les capteurs sont de préférence au nombre de 4, et situés aux extrémités de la plateforme par exemple séparé de 20 à 50 cm pour les capteurs droite et gauche ou haut et bas. Par exemple, comme cela est présenté en figure 6, la plateforme 310 comporte quatre capteurs 312 situés aux quatre coins du plateau (Haut gauche, Haut droit, Bas gauche, Bas droit).

La plateforme 310 comporte avantageusement un module de décompte du temps et peut être configurée pour mesurer les valeurs de ses différents capteurs 312 à intervalle aléatoire, à une fréquence pouvant varier par exemple de 10 Hz à 1000 Hz. De préférence, la plateforme 310 est configurée pour mesurer les valeurs de ses différents capteurs 312 à une fréquence supérieure ou égale à 25 Hz, de façon plus préférée supérieure ou égale à 50 Hz.

De façon encore plus préférée, la plateforme 310 est configurée pour mesurer, lors de l'acquisition d'un statokinésigramme 110, les valeurs de ses différents capteurs 312 à une fréquence supérieure ou égale à 25 Hz et de façon sensiblement constante. En effet, si la fréquence d'échantillonnage est trop faible, ou trop aléatoire, la quantification de l'équilibre ne sera pas suffisamment précise. Si la fréquence n'est pas constante alors de façon préférée la fréquence moyenne d'acquisition est supérieure ou égale à 60 Hz, de façon plus préférée supérieure ou égale à 75 Hz.

La plateforme 310 peut comporter un dispositif d'affichage, de préférence positionné de sorte que l'individu, debout sur le plateau 311 puisse voir le dispositif d'affichage.

La plateforme 310 peut également comporter un dispositif de haut-parleur pouvant donner des instructions à l'individu (e.g. monter ou descendre du plateau 311). Ces instructions peuvent également être données par le dispositif d'affichage.

Avantageusement, il est possible d'augmenter la sensibilité de la quantification par l'utilisation d'une mousse posée sur la plateforme et apte à déformer ou perturber les informations proprioceptives et tactiles. Cette mousse peut par exemple présenter une épaisseur de 1 à 10 millimètres et une densité comprise entre 100 et 500 kg/m$^3$.

La plateforme 310 peut également comporter un module de mesure du poids de l'individu, de sa masse grasse, hydrique, osseuse, musculaire, de sa fréquence cardiaque et/ou de son indice de masse corporelle.

Le système de quantification de l'équilibre 3 comporte également **une unité de traitement des données brutes 320** générées par la plateforme. Cette unité de traitement des données brutes 320 est agencée et/ou configurée pour générer au moins un statokinésigramme 110 de l'individu, à partir des données brutes générées par les capteurs 312. Cette unité de traitement des données brutes 320 peut être par exemple intégrée à la plateforme 310 comme cela est représenté dans la figure 6. Néanmoins, elle peut également être intégrée à un serveur distant 330, au dispositif de quantification 2 (e.g. elle y intègre alors le module de génération 230 de statokinésigramme 110) ou à un dispositif de commande 340.

Le système de quantification de l'équilibre 3 peut comporter un **serveur distant 330** comme cela est représenté dans la figure 6. Il est par exemple possible d'accéder à ce serveur distant 330 via une interface web ou directement via les fonctions appropriées directement implémentées sur un dispositif de commande 340. Toutes les communications entre le ou les dispositif(s) de commande 340 et le serveur distant 330 peuvent être sécurisées par exemple par des protocoles HTTPS et cryptage AES 512.

Ce serveur distant peut héberger le dispositif de quantification 2. Ainsi, un seul dispositif de quantification 2 peut suivre une pluralité d'individus.

Le système de quantification selon l'invention peut comprendre un **dispositif de commande 340** du système configuré pour interagir avec la plateforme 310 et le dispositif de quantification de l'équilibre 2. Ce dispositif de commande 340 du système permet par exemple de piloter l'acquisition des données à partir de la plateforme 310 et d'afficher les résultats en provenance du dispositif de quantification 2.

Ce dispositif de commande 340 du système est de présence un dispositif mobile tel qu'une tablette 340a, un ordinateur portable, ou une montre.

Selon un **autre aspect, l'invention porte sur un produit programme d'ordinateur 4** configuré pour mettre en oeuvre le procédé de quantification 1 de l'équilibre selon l'invention. Le produit programme d'ordinateur 4 est enregistré sur un support mémoire non transitoire et est apte à être exécuté sur un ordinateur, une tablette ou un serveur ; ledit programme d'ordinateur comportant au moins :

- un algorithme adapté pour segmenter un statokinésigramme 110 d'un individu de façon à générer plusieurs portions de statokinésigramme,
- un algorithme adapté pour extraire, à partir des portions 120 de statokinésigramme des valeurs d'au moins un paramètre de trajectoire 130,
- un algorithme adapté pour déterminer plusieurs quantificateurs 140, à partir des valeurs des paramètres de trajectoire 130 extraites, et
- un algorithme adapté pour déterminer une valeur représentative de l'équilibre de l'individu à partir des valeurs desdits quantificateurs 140 de chacune des portions 120 de statokinésigramme.

Le procédé, le dispositif, le système et le produit programme d'ordinateur selon l'invention permettent la quantification de l'équilibre d'un individu et peuvent avoir de **nombreuses applications.** En effet, l'invention permet d'apporter un outil de mesure, à savoir un procédé, le dispositif de mise en oeuvre du procédé et le système intégrant le dispositif, qui permet d'obtenir une valeur chiffrée et objective de l'équilibre d'un individu afin de répondre à plusieurs questions principales relatives à l'équilibre d'un individu telles que l'évolution de l'équilibre, naturelle ou sous traitement, la qualité de l'équilibre et donc, son corollaire, la sévérité d'un éventuel trouble de l'équilibre (e.g. quel est le risque de chute ?), et la ou les causes de l'éventuel trouble de l'équilibre.

En effet, la génération d'une valeur représentative de l'équilibre d'un individu et indicative de la qualité de son équilibre permet à l'individu, ou à d'autres personnes, d'attribuer une valeur chiffrée et objective à cet équilibre.

Ces valeurs ou ces scores peuvent être utilisés dans le cadre d'un suivi dans le temps visant à identifier des déviations par rapport à la référence apprise.

De même, l'invention peut être utilisée pour mettre en évidence les effets de différents traitements et le taux de récupération pourrait être suivi par la quantification de l'équilibre selon l'invention. Ainsi, l'invention peut être mise en oeuvre dans le cadre de l'évaluation de la performance de programme sportif, de prothèses, de chaussure de sport, de semelles de compensation, de protocoles de rééducation, de traitement des désordres neurologiques et/ou de techniques de chirurgie. Le procédé selon l'invention est particulièrement adapté aux personnes âgées

En outre, l'invention peut être utilisée pour comparer la qualité de l'équilibre d'un individu avec la qualité de l'équilibre d'autres individus et déterminer par exemple si cet individu présente un risque de chute. Ainsi, l'invention peut être mise en oeuvre dans le cadre de la mesure d'un risque de chute par exemple à 6 mois. Dans ce contexte, la valeur représentative de l'équilibre déterminée par le procédé de quantification 1 selon l'invention, une valeur indicative d'un risque de chute à 6 mois. De façon particulière, l'individu au sens de l'invention est une personne de plus de 60 ans, de préférence de plus de 70 ans.

De plus, la comparaison des valeurs des quantificateurs 140 obtenues chez un individu à des valeurs des quantificateurs obtenues chez différentes catégories de personnes peut permettre de cibler la prise en charge des individus et de les orienter vers les services adaptés (e.g. traumatologie, rhumatologie, neurologie). Ainsi, l'invention peut être mise en oeuvre dans le cadre de la détermination de l'origine du trouble de l'équilibre.

**Exemples**

individus étudiés

Les résultats présentés ci-dessous ont été réalisés selon un protocole approuvé par l'Agence Nationale de Sécurité du Médicament et des produits de santé et un consentement écrit a été obtenu pour l'ensemble des participants. L'invention a été mise en oeuvre sur un groupe de 126 individus présentant les caractéristiques suivantes :

- âgés de plus de 65 ans,
- en bonne santé (e.g. patients ayant de l'hypertension ont été exclus)
- capables de se tenir debout sur la plate-forme,
- ayant donné un consentement éclairé.

Parmi les 126 individus inclus dans l'étude, 18 ont déclaré une chute au cours des 6 mois précédant la consultation.

<u>Mesure du déplacement du centre de pression</u>

Lors de la consultation, les déplacements du centre de pression des individus ont été suivis grâce à une Wii Balance Board (marque déposée) et enregistrés grâce à une application personnalisée spécialement développée dans le cadre de l'invention. Les pieds ont été positionnés dans la position la plus confortable pour le patient, sans dépasser la largeur des épaules. La trajectoire du centre de pression a été enregistrée pendant 25 secondes les yeux ouverts, puis 25 secondes les yeux fermés. Un questionnaire de chute a été rempli pour chaque individu afin d'enregistrer les chutes déclarées s'étant produites au cours des 6 derniers mois.

<u>Pré-traitement</u>

Avant de calculer les statokinésigrammes, la sortie des signaux bruts par le WBB ont été dé-bruités et ré-échantillonnés.

<u>Segmentation</u>

Les statokinésigrammes ont ensuite été segmentés en portion de 1 à 3 secondes avec ou sans recouvrement.

<u>Analyse statistique selon l'invention</u>

Pour chaque portion de statokinésigramme, trois quantificateurs ont été calculées : la surface de l'ellipse de confiance à 95 %, la moyenne de la norme de la vitesse de déplacement du centre de pression, la variance des valeurs de la position du centre de pression selon l'axe médio-latéral. Ces quantificateurs ont alors été normalisés.
Deux modèles de mélange gaussien (GMM) ont été utilisés, un pour les yeux ouverts, le second pour les yeux fermés. Les modèles de mélange gaussien (GMM) sont des algorithmes de partitionnement non supervisé.
Considérant un certain nombre de catégorie k (dans cet exemple, k = 2 pour déplacement du centre de pression régulier-RP ou irrégulier-IP), et un ensemble de points Zj (ici chaque point Zj correspond aux valeurs de quantificateurs normalisés pour chacune des portions), le GMM essaie de construire un mélange de 2 (k) variables Gaussienne aléatoire multivarié, suivant le principe de la vraisemblance maximale. Pour ce faire, il procède avec selon le principe de l'algorithme d'espérance-maximisation (EM) de façon à maximiser la loi de vraisemblance en présence de données incomplètes en maximisant itérativement l'espérance de la log-vraisemblance.
Les poids $\pi_i$, les centres $(\mu_i)^k_{i=1}$ et la matrice de covariance matrice de chaque variable aléatoire gaussienne $N_i$ sont initialisés de manière aléatoire en utilisant la procédure classique itérative (e.g. 20 fois). Ensuite, l'algorithme répète les étapes suivantes jusqu'à la convergence
La probabilité $p_{i,j}$ que le point $Z_j$ appartient au groupe i :

$$p_{i,j} = \frac{\frac{\pi_i}{\sqrt{|\Sigma_i|}} \exp\left(-(Z_j - \mu_i)^T \Sigma_i^{-1}(Z_j - \mu_i)\right)}{\sum_i \frac{\pi_i}{\sqrt{|\Sigma_i|}} \exp\left(-(Z_j - \mu_i)^T \Sigma_i^{-1}(Z_j - \mu_i)\right)}$$

Les poids $\pi_i$, les centres $(\mu_i)^k_{i=1}$ et la matrice de covariance sont mis à jour en fonction de la probabilité $p_{i,j}$

$$\pi_i = \frac{1}{n} \sum_j p_{i,j}$$

$$\mu_i = \frac{1}{\sum_j p_{i,j}} \sum_j p_{i,j} Z_j$$

$$\Sigma_i = \frac{1}{\sum_j p_{i,j}} \sum_j p_{i,j}(Z_j - \mu_i)^T(Z_j - \mu_i)$$

Cela permet de calculer un score à partir des résultats obtenus pour chaque portion.

Analyse statistique comparative, Ondelette

Dans la littérature, il est mentionné que dans le cadre d'analyse en ondelette, chaque bande de fréquence peut être liée à différentes entrées sensorielles. Ainsi, l'étude de la distribution d'énergie pourrait permettre de différentier différents équilibres. Ainsi, trois bandes de fréquence ont été étudiées sur cet échantillon de façon à analyser la distribution de l'énergie dans ces bandes. Comme dans l'analyse proposée selon l'invention, la performance finale est vérifiée en faisant la moyenne des scores de chaque portion et en créant un score global. Le score final dans l'analyse des ondelettes est le pourcentage d'énergie contenu dans ces bandes. Une telle analyse permet d'obtenir un résultat d'AUC compris entre 0,48 et 0,52, soit un résultat aléatoire.

Ainsi, la méthode des ondelettes ne parait pas adaptée aux problématiques adressées par la présente invention.

Résultats de l'invention

Dans le tableau 1 ci-dessous sont présentés les résultats d'AUC en fonction des paramètres appliqués au procédé et en comparaison avec une analyse réalisée sans segmentation.

Ces résultats montrent qu'avec les quantificateurs sélectionnés et en l'absence de segmentation, les résultats d'AUC sont de 0,63 avec une déviation standard de 0,12.

Au contraire, en présence d'une segmentation, les résultats d'AUC sont au minimum de 0,75, soit une augmentation minimale de 19 %, et la déviation standard est plus faible pour toutes les conditions testées. Ainsi, la mise en oeuvre du procédé selon l'invention permet d'augmenter la performance de la quantification de l'équilibre et d'en réduire la variabilité.

En outre, en présence d'un recouvrement de 50 %, l'AUC peut être encore augmenté avec, pour des portions d'une seconde, un AUC de 0,77.

Tableau 1 :

| | Recouvrement de 50 % | Sans recouvrement |
|---|---|---|
| Durée de chaque portion | AUC (déviation) | AUC (déviation) |
| 1 sec | 0,77(0,09) | 0,75(0,11) |
| 2 sec | 0,77(0,10) | 0,75(0,11) |
| 3 sec | 0,76(0,09) | 0,75(0,08) |
| Sans segmentation | na | 0,63(0,12) |

Ainsi, l'invention apporte un outil de mesure permettant d'obtenir une quantification améliorée de l'équilibre d'un individu. Cette quantification peut notamment prendre en compte des signaux faibles, des déséquilibres transitoires qui pourraient être masqués lors de l'analyse de statokinésigrammes complets. En outre, l'invention permet de faciliter l'appréciation de l'équilibre d'un individu faite par l'intermédiaire de représentations graphiques permettant de qualifier chaque portion d'un statokinésigramme. Avantageusement, un utilisateur pourra facilement naviguer dans ce jeu de données et pourra identifier le cas échéant des motifs caractéristiques de troubles de l'équilibre.

Tous ces avantages contribuent à améliorer la quantification de l'équilibre d'un individu.

**Revendications**

1. Procédé de quantification (1) de l'équilibre d'un individu pour obtenir une valeur représentative de l'équilibre dudit individu, ledit procédé étant mis en oeuvre par un dispositif comprenant au moins un module de traitement de données (220) connecté à un moyen de mémorisation (280), ledit procédé comprenant un enregistrement (10), sur le moyen de mémorisation (280), d'au moins un statokinésigramme de l'individu (110) obtenu à partir d'une plateforme (310) comprenant des capteurs (312) de pression et/ou de force, ledit procédé comprenant en outre les étapes suivantes :

   - extraction (30), par le module de traitement de données (220) et à partir des statokinésigramme(s), des valeurs d'au moins un paramètre de trajectoire (130),
- détermination (40), par le module de traitement de données (220), de la valeur d'au moins deux quantificateurs (140), à partir des valeurs de paramètres de trajectoire (130) extraites à l'étape d'extraction (30) et
- détermination (50), par le module de traitement de données (220), de ladite valeur représentative de l'équilibre de l'individu à partir des valeurs des quantificateurs,
**caractérisé en ce que** le procédé présente préalablement à l'étape d'extraction une étape de segmentation (20), en fonction du temps, du ou des statokinésigramme(s) de l'individu (110) de façon à générer plusieurs portions (120) de statokinésigramme(s),
et **en ce que** dans le procédé :

- l'extraction (30) des valeurs d'au moins un paramètre de trajectoire (130) est réalisée à partir des portions de statokinésigramme(s) (120),
- la détermination (40), de la valeur d'au moins deux quantificateurs (140), à partir des valeurs de paramètres de trajectoire (130) extraites à l'étape d'extraction (30) est réalisée pour chacune des portions (120) de statokinésigramme(s) générées à l'étape de segmentation (20), et
- la détermination (50) de ladite valeur représentative de l'équilibre de l'individu est réalisée à partir des valeurs des quantificateurs de chacune des portions (120) de statokinésigramme(s).

2. Procédé de quantification selon la revendication 1, **caractérisé en ce que** les portions (120) de statokinésigramme(s) présentent une durée inférieure ou égale à trois secondes.

3. Procédé de quantification selon l'une des revendications 1 ou 2, **caractérisé en ce que** l'étape de segmentation (20) génère, pour chaque statokinésigramme, au moins dix portions (120) de statokinésigramme.

4. Procédé de quantification selon l'une quelconque des revendications 1 à 3, **caractérisé en ce que** les portions (120) de statokinésigramme générées lors de l'étape de segmentation (20) présentent, pour des portions consécutives, un taux de recouvrement d'au moins 25%, de préférence d'au moins 50 %.

5. Procédé de quantification selon l'une quelconque des revendications 1 à 4, **caractérisé en ce que** les portions (120) de statokinésigramme générées lors de l'étape de segmentation (20) présentent, pour des portions consécutives, un taux de recouvrement au plus de 95 %.

6. Procédé de quantification selon l'une quelconque des revendications 1 à 5, **caractérisé en ce que** l'étape de détermination (50) est réalisée en mettant en oeuvre les valeurs des quantificateurs (140) déterminées à l'étape de détermination (40) dans un algorithme de notation de façon, en fonction des valeurs des quantificateurs (140), à attribuer un score à chacune des portions (120) de statokinésigrammes ou à classer chacune des portions (120) de statokinésigrammes par catégories, de préférence, l'algorithme de notation est un algorithme de partitionnement non supervisé.

7. Procédé de quantification selon la revendication précédente, **caractérisé en ce que** l'algorithme de partitionnement non supervisé est sélectionné parmi un modèle de mélange Gaussien non supervisé, une classification ascendante hiérarchique ou une classification descendante hiérarchique.

8. Procédé de quantification selon l'une des revendications précédentes, caractérisé en ce le procédé comprend en outre :

- une étape de génération (11) de données brutes correspondant au déplacement du centre d'une pression appliquée par l'ensemble du corps d'un individu dans le temps sur une plateforme ;
- une étape de transformation (12) des données brutes en données de trajectoire du centre de pression.

les données brutes correspondant au déplacement du centre de pression d'un individu sont obtenues lors d'un test de Romberg, de préférence l'étape de segmentation (20) est réalisée à partir d'un statokinésigramme (110) obtenu alors que l'individu a les yeux ouverts et d'un statokinésigramme (110) obtenu lorsque l'individu a les yeux fermés.

9. Procédé de quantification selon la revendication précédente, **caractérisé en ce que** le procédé comporte, pour au moins un quantificateur, le calcul d'un rapport O/F ou F/O correspondant au rapport entre la valeur d'un paramètre de trajectoire calculé à partir d'un statokinésigramme (110) obtenu alors que l'individu a les yeux ouverts et la valeur d'un paramètre de trajectoire calculé à partir d'un statokinésigramme (110) obtenu alors que l'individu a les yeux fermés (rapport O/F) ou l'inverse (rapport F/O).

10. Procédé de quantification selon l'une des revendications précédentes, **caractérisé en ce que** le paramètre de trajectoire (130) est un paramètre de trajectoire de position (131) du centre de pression, de stabilité (132) du centre de pression, et/ou dynamique (133) du centre de pression.

11. Dispositif (2) de quantification de l'équilibre d'un individu, ledit dispositif comprenant :

    - un module de communication (210) apte à recevoir un statokinésigramme (110) dudit individu,
    - un moyen de mémorisation (280) apte à enregistrer ledit statokinésigramme, et
    - au moins un module de traitement de données (220), apte à se connecter au moyen de mémorisation (280),

    ledit module de traitement de données (220) étant configuré pour :

    o Extraire, à partir de statokinésigramme, des valeurs d'au moins un paramètre de trajectoire (130),
    o Déterminer plusieurs quantificateurs (140), à partir des valeurs de paramètre de trajectoire (130) extraites,
    o Déterminer une valeur représentative de l'équilibre de l'individu à partir des valeurs desdits quantificateurs (140) des statokinésigrammes,
    le dispositif étant **caractérisé en ce que** ledit module de traitement de données est configuré pour avoir, préalablement à l'étape d'extraction, une étape de segmentation, en fonction du temps, du statokinésigramme (110) de l'individu enregistré sur le moyen de mémorisation de façon à générer plusieurs portions (120) de statokinésigramme,
    et **en ce que** le module de traitement de données est configuré pour :

    - Extraire, à partir des portions (120) de statokinésigramme, des valeurs d'au moins un paramètre de trajectoire (130), et
    - Déterminer une valeur représentative de l'équilibre de l'individu à partir des valeurs desdits quantificateurs (140) de chacune des portions (120) de statokinésigrammes.

12. Dispositif (2) de quantification selon la revendication précédente, **caractérisé en ce que** le dispositif comporte en outre un module de ré-échantillonnage (240) configuré pour traiter les données brutes ou les statokinésigramme (110) à une première fréquence de façon à générer des statokinésigrammes (110) ré-échantillonnés à une seconde fréquence et présentant une fréquence sensiblement constante.

13. Système (3) de quantification de l'équilibre d'un individu, comprenant :

    - une plateforme (310), ladite plateforme (310) étant adaptée à recevoir un individu et comprenant des capteurs (312) de pression et/ou de force configurés pour générer des données brutes, à une première fréquence, fonction d'une pression exercée par les pieds de l'individu sur la plateforme (310),
    - une unité de traitement des données brutes (320), agencée pour obtenir au moins un statokinésigramme (110) de l'individu à partir des données brutes générées par la plateforme, et
    - un dispositif (2) de quantification de l'équilibre selon la revendication 11 ou 12, apte à communiquer avec l'unité de traitement.

14. Système (3) de quantification selon la revendication précédente, **caractérisé en ce que** la plateforme comporte quatre capteurs (312) de pression et/ou de force.

15. Produit programme d'ordinateur (4) comprenant des instructions qui, lorsque le programme est exécuté sur un ordinateur, conduisent celui-ci à mettre en oeuvre le procédé selon les revendications 1 à 10.

**Patentansprüche**

1. Verfahren zur Quantifizierung (1) des Gleichgewichts einer Person, um einer repräsentativen Wert des Gleichgewichts der Person zu erhalten, wobei das Verfahren durch eine Vorrichtung durchgeführt wird, die mindestens ein Datenverarbeitungsmodul (220) umfasst, das mit einem Speichermittel (280) verbunden ist, wobei das Verfahren eine Aufzeichnung (10) von mindestens einem Statokinesigramm der Person (110) auf dem Speichermittel (280) umfasst, das aus einer Plattform (310) erhalten wird, die Druck- und/oder Kraftsensoren (312) umfasst, wobei das Verfahren ferner die folgenden Schritte umfasst:

- Extrahieren (30) von Werten von mindestens einem Wegparameter (130) durch das Datenverarbeitungsmodul (220) und aus einem oder mehreren Statokinesigrammen,
- Bestimmen (40) des Werts von mindestens zwei Quantifikatoren (140) durch das Datenverarbeitungsmodul (220) aus Werten von Wegparametern (130), die in dem Extraktionsschritt (30) extrahiert wurden, und
- Bestimmen (50) des repräsentativen Werts des Gleichgewichts der Person durch das Datenverarbeitungsmodul (220) aus Werten von Quantifikatoren,
**dadurch gekennzeichnet, dass** das Verfahren vor dem Extraktionsschritt einen Schritt eines Segmentierens (20) des oder der Statokinesigramme der Person (110) in Abhängigkeit von der Zeit aufweist, um mehrere Abschnitte (120) eines oder mehrerer Statokinesigramme zu erzeugen,
und dass in dem Verfahren:

- das Extrahieren (30) von Werten von mindestens einem Wegparameter (130) aus Abschnitten eines oder mehrerer Statokinesigramme (120) umgesetzt wird,
- das Bestimmen (40) des Werts von mindestens zwei Quantifikatoren (140) aus Werten von Wegparametern (130), die in dem Extraktionsschritt (30) extrahiert wurden, für jeden von Abschnitten (120) eines oder mehrerer Statokinesigramme, die in dem Segmentierungsschritt (20) erzeugt wurden, umgesetzt wird, und
- das Bestimmen (50) des repräsentativen Werts des Gleichgewichts der Person aus Werten von Quantifikatoren von jedem von Abschnitten (120) eines oder mehrerer Statokinesigramme umgesetzt wird.

2. Quantifizierungsverfahren nach Anspruch 1, **dadurch gekennzeichnet, dass** die Abschnitte (120) eines oder mehrerer Statokinesigramme eine Dauer aufweisen, die kürzer als oder gleich drei Sekunden ist.

3. Quantifizierungsverfahren nach einem der Ansprüche 1 oder 2, **dadurch gekennzeichnet, dass** der Segmentierungsschritt (20) für jedes Statokinesigramm mindestens zehn Abschnitte (120) eines Statokinesigramms erzeugt.

4. Quantifizierungsverfahren nach einem der Ansprüche 1 bis 3, **dadurch gekennzeichnet, dass** die Abschnitte (120) eines Statokinesigramms, die während des Segmentierungsschritts (20) erzeugt werden, für aufeinanderfolgende Abschnitte einen Überlappungsgrad von mindestens 25%, vorzugsweise mindestens 50% aufweisen.

5. Quantifizierungsverfahren nach einem der Ansprüche 1 bis 4, **dadurch gekennzeichnet, dass** die Abschnitte (120) eines Statokinesigramms, die während des Segmentierungsschritts (20) erzeugt werden, für aufeinanderfolgende Abschnitte einen Überlappungsgrad von höchstens 95% aufweisen.

6. Quantifizierungsverfahren nach einem der Ansprüche 1 bis 5, **dadurch gekennzeichnet, dass** der Bestimmungsschritt (50) durch Einsetzen von Werden von Quantifikatoren (140), die in dem Bestimmungsschritt (40) bestimmt wurden, in einen Bewertungsalgorithmus umgesetzt wird, um in Abhängigkeit von Werten von Quantifikatoren (140) jedem von Abschnitten (120) von Statokinesigrammen eine Punktezahl zuzuordnen oder jeden von Abschnitten (120) von Statokinesigrammen durch Kategorien zu klassifizieren, vorzugsweise wobei der Bewertungsalgorithmus ein nicht überwachter Aufteilungsalgorithmus ist.

7. Quantifizierungsverfahren nach dem vorhergehenden Anspruch, **dadurch gekennzeichnet, dass** der nicht überwachte Aufteilungsalgorithmus aus einem nicht überwachten Gaußschen Mischmodell, einer hierarchischen aufsteigenden Klassifizierung oder einer hierarchischen absteigenden Klassifizierung ausgewählt ist.

8. Quantifizierungsverfahren nach einem der vorhergehenden Ansprüche, **dadurch gekennzeichnet, dass** das Verfahren ferner umfasst:

- einen Schritt eines Erzeugens (11) von Rohdaten, die der Verschiebung des Zentrums eines Drucks, der von dem gesamten Körper der Person während der Zeit auf der Plattform ausgeübt wird, entsprechen;
- einen Schritt eines Umwandelns (12) von Rohdaten in Wegdaten des Druckzentrums,

wobei die Rohdaten, die der Verschiebung des Zentrums eines Drucks einer Person entsprechen, während eines Romberg-Tests erhalten werden, vorzugsweise wobei der Segmentierungsschritt (20) aus einem Statokinesigramm (110), das erhalten wurde, als die Person die Augen offen hielt, und einem Statokinesigramm (110), das erhalten wurde, als die Person die Augen geschlossen hielt, umgesetzt wird.

9. Quantifizierungsverfahren nach dem vorhergehenden Anspruch, **dadurch gekennzeichnet, dass** das Verfahren für mindestens einen Quantifikator das Berechnen eines O/G- oder G/O-Verhältnisses umfasst, das dem Verhältnis zwischen dem Wert eines Wegparameters, der aus einem Statokinesigramm (110), das erhalten wurde, als die Person die Augen offen hielt, berechnet wurde, und dem Wert eines Wegparameters, der aus einem Statokinesigramm (110), das erhalten wurde, als die Person die Augen geschlossen hielt, berechnet wurde, (O/G-Verhältnis) oder umgekehrt (G/O-Verhältnis) entspricht.

10. Quantifizierungsverfahren nach einem der vorhergehenden Ansprüche, **dadurch gekennzeichnet, dass** der Wegparameter (130) ein Wegparameter einer Position (131) des Druckzentrums, einer Stabilität (132) des Druckzentrums und/oder ein dynamischer Wegparameter (133) des Druckzentrums ist.

11. Vorrichtung (2) zur Quantifizierung des Gleichgewichts einer Person, wobei die Vorrichtung umfasst:

   - ein Kommunikationsmodul (210), das dazu fähig ist, ein Statokinesigramm (110) der Person zu empfangen,
   - ein Speichermittel (280), das dazu fähig ist, das Statokinesigramm aufzuzeichnen, und
   - mindestens ein Datenverarbeitungsmodul (220), das dazu fähig ist, sich mit dem Speichermittel (280) zu verbinden,

   wobei das Datenverarbeitungsmodul (220) konfiguriert ist zum:

   ◦ Extrahieren von Werten von mindestens einem Wegparameter (130) aus einem Statokinesigramm,
   ◦ Bestimmen von mehreren Quantifikatoren (140) aus extrahierten Werten eines Wegparameters (130),
   ◦ Bestimmen eines repräsentativen Werts des Gleichgewichts der Person aus Werten der Quantifikatoren (140) von Statokinesigrammen,
   wobei die Vorrichtung **dadurch gekennzeichnet ist, dass** das Datenverarbeitungsmodul dazu konfiguriert ist, vor dem Extraktionsschritt einen Schritt eines Segmentierens des Statokinesigramms (110) der Person, das auf dem Speichermittel aufgezeichnet ist, in Abhängigkeit von der Zeit aufzuweisen, um mehrere Abschnitte (120) eines Statokinesigramms zu erzeugen,
   und dass das Datenverarbeitungsmodul konfiguriert ist zum:

   - Extrahieren von Werten von mindestens einem Wegparameter (130) aus Abschnitten (120) eines Statokinesigramms und
   - Bestimmen eines repräsentativen Werts des Gleichgewichts der Person aus Werten der Quantifikatoren (140) von jedem von Abschnitten (120) von Statokinesigrammen.

12. Quantifizierungsvorrichtung (2) nach dem vorhergehenden Anspruch, **dadurch gekennzeichnet, dass** die Vorrichtung ferner ein Neuabtastungsmodul (240) umfasst, das dazu konfiguriert ist, die Rohdaten oder die Statokinesigramme (110) mit einer ersten Frequenz zu verarbeiten, um neu abgetastete Statokinesigramme (110) mit einer zweiten Frequenz zu erzeugen, die eine im Wesentlichen konstante Frequenz aufweisen.

13. System (3) zur Quantifizierung des Gleichgewichts einer Person, umfassend:

   - eine Plattform (310), wobei die Plattform (310) dazu angepasst ist, eine Person aufzunehmen, und Druck- und/oder Kraftsensoren (312) umfasst, die dazu konfiguriert sind, Rohdaten mit einer ersten Frequenz in Abhängigkeit von einem Druck, der von den Füßen der Person auf der Plattform (310) ausgeübt wird, zu erzeugen,
   - eine Rohdatenverarbeitungseinheit (320), die dazu eingerichtet ist, mindestens ein Statokinesigramm (110) der Person aus Rohdaten, die von der Plattform erzeugt wurden, zu erhalten, und
   - eine Vorrichtung (2) zur Quantifizierung des Gleichgewichts nach Anspruch 11 oder 12, die dazu fähig ist, mit der Verarbeitungseinheit zu kommunizieren.

14. Quantifizierungssystem (3) nach dem vorhergehenden Anspruch, **dadurch gekennzeichnet, dass** die Plattform vier Druck- und/oder Kraftsensoren (312) umfasst.

15. Computerprogrammprodukt (4), das Anweisungen umfasst, die, wenn das Programm von einem Computer ausgeführt wird, bewirken, dass dieses das Verfahren nach einem der Ansprüche 1 bis 10 durchführt.

**Claims**

1. A method (1) for quantifying the balance of an individual to obtain a value representative of the balance of said individual, said method being implemented by a device comprising at least one data processing module (220) connected to a storage means (280), said method comprising recording (10), on the storage means (280), of at least one statokinesigram (110) of the individual obtained from a platform (310) comprising pressure and/or force sensors (312), said method further comprising the following steps:

   - extracting (30), by the data processing module (220) and from the statokinesigram(s), the values of at least one trajectory parameter (130),
   - determining (40), by the data processing module (220), the value of at least two quantifiers (140), from the values of trajectory parameters (130) extracted in the extraction step (30), and
   - determining (50), by the data processing module (220), said value representative of the balance of the individual from the values of the quantifiers.

   **characterized in that** the method presents, prior to the extraction step, a segmentation (20) step, as a function of time, of the statokinesigram(s) (110) of the individual so as to generate several portions (120) of statokinesigram(s),
   and **in that** in the method:

      - the extraction (30), of the values of at least one trajectory parameter (130) is carried out from the portions (120) of statokinesigram(s),
      - the determination (40), of the value of at least two quantifiers (140), from the values of trajectory parameters (130) extracted in the extraction step (30), is carried out for each of the portions (120) statokinesigram(s) generated in the segmentation step (20), and
      - the determination (50) of said value representative of the balance of the individual is carried out from the values of the quantifiers of each of the portions (120) of statokinesigram(s).

2. The quantification method according to claim 1, **characterized in that** the statokinesigram(s) portions (120) have a duration of less than or equal to three seconds.

3. The quantification method according to one of claims 1 or 2, **characterized in that** the segmentation step (20) generates, for each statokinesigram, at least ten statokinesigram portions (120).

4. The quantification method according to any one of claims 1 to 3, **characterized in that** the statokinesigram portions (120) generated during the segmentation step (20) have, for consecutive portions, an overlap ratio of at least 25%, preferably at least 50%.

5. The quantification method according to any one of claims 1 to 4, **characterized in that** the statokinesigram portions (120) generated during the segmentation step (20) have, for consecutive portions, an overlap ratio of at most 95%.

6. The quantification method according to any one of claims 1 to 5, **characterized in that** the determination step (50) is carried out by implementing the values of the quantifiers (140) determined in the determination step (40) in a scoring algorithm in order to, depending on the values of the quantifiers (140), assign a score to each of the statokinesigram portions (120) or classify each of the statokinesigram portions (120) by categories, preferably, the scoring algorithm is an unsupervised partitioning algorithm.

7. The quantification method according to the preceding claim, **characterized in that** the unsupervised partitioning algorithm is selected from an unsupervised Gaussian mixture model, a hierarchical bottom-up classification, or a hierarchical top-down classification.

8. The quantification method according to one of the preceding claims, **characterized in that** the method further comprises:

   - a step of generating (11) raw data corresponding to the displacement of the center of a pressure applied by the whole body of an individual over time on a platform;
   - a step of transforming (12) the raw data into trajectory data of the center of pressure;
   - the raw data corresponding to the displacement of the center of pressure of an individual are obtained in a Romberg test, preferably the segmentation step (20) is performed from a statokinesigram (110) obtained while the individual has his/her eyes open and a statokinesigram (110) obtained while the individual has his/her eyes closed.

9. The quantification method according to the preceding claim, **characterized in that** the method includes, for at least one quantifier, calculating an O/F or F/O ratio corresponding to the ratio between the value of a trajectory parameter calculated from a statokinesigram (110) obtained while the individual has his/her eyes open and the value of a trajectory parameter calculated from a statokinesigram (110) obtained while the individual has his/her eyes closed (O/F ratio) or the opposite (F/O ratio).

10. The quantification method according to one of the preceding claims, **characterized in that** the trajectory parameter (130) is a position trajectory parameter (131), stability trajectory parameter (132), and/or dynamics trajectory parameter (133) trajectory parameter of the center of pressure.

11. A device (2) for quantifying the balance of an individual, said device comprising:

 - a communication module (210) able to receive a statokinesigram (110) of said individual,
 - a storage means (280) able to record said statokinesigram, and
 - at least one data processing module (220), able to connect to the storage means (280),
 said data processing module (220) being configured to:

 o Extract, from the statokinesigram, values of at least one trajectory parameter (130),
 o Determine several quantifiers (140), from the extracted values of trajectory parameters (130),
 o Determine a value representative of the balance of the individual from the values of said quantifiers (140) of each of the statokinesigram,

 said device being **characterized in that** said data processing module is configured to have, prior to the extraction step, a step of segmenting, as a function of time, the statokinesigm (110) of the individual recorded on the storage means so as to generate several portions (120) of statokinesigm(s);
 and **in that** the data processing module is configured to :

 - extracting, from the statokinesigram portions (120), values of at least one trajectory parameter (130), and
 - determining a value representative of the balance of the individual from the values of said quantifiers (140) of each of the statokinesigram(s) portions (120).

12. The quantification device (2) according to the preceding claim, **characterized in that** the device further comprises a re-sampling module (240) configured to process the raw data or the statokinesigrams (110) at a first frequency so as to generate re-sampled statokinesigrams (110) at a second frequency and having a substantially constant frequency.

13. A system (3) for quantifying the balance of an individual, comprising:

 - a platform (310), said platform (310) being adapted to receive an individual and comprising pressure and/or force sensors (312) configured to generate raw data, at a first frequency, as a function of a pressure exerted by the feet of the individual on the platform (310),
 - a raw data processing unit (320), arranged to obtain at least one statokinesigram (110) of the individual from the raw data generated by the platform, and
 - a balance quantification device (2) according to claim 11 or 12, able to communicate with the processing unit.

14. The quantification system (3) according to the preceding claim, **characterized in that** the platform includes four pressure and/or force sensors (312).

15. A computer program product (4) comprising instructions which, when the program is executed on a computer, cause the computer to implement the method of claims 1 to 10.

1

10 — Enregistrement d'au moins un statokinésigramme de l'individu obtenu à partir d'une plateforme comprenant des capteurs de pression et/ou de force

20 — Segmentation en fonction du temps, du ou des statokinésigramme(s) de l'individu enregistré(s) sur le moyen de mémorisation de façon à générer plusieurs portions de statokinésigramme(s)

30 — Extraction à partir des portions de statokinésigramme(s), des valeurs d'au moins un paramètre de trajectoire

40 — Détermination de la valeur d'au moins deux quantificateurs, à partir des valeurs de paramètres de trajectoire extraites à l'étape d'extraction pour chacune des portions de statokinésigramme générées à l'étape de segmentation

50 — Détermination de ladite valeur représentative de l'équilibre de l'individu à partir des valeurs des quantificateurs de chacune des portions de statokinésigramme

60 — Enregistrement de la valeur représentative de l'équilibre obtenue

70 — Transmission du score ou de la catégorie de chacune des portions de statokinésigramme de façon à permettre la génération d'une représentation graphique des portions du statokinésigramme en fonction dudit score ou de ladite catégorie.

**FIG. 1**

**FIG. 2A**

**FIG. 2B**

**FIG. 2C**

**FIG. 2D**

**FIG. 2E**

**FIG. 2F**

**FIG. 3**

**FIG. 4**

**FIG. 5**

**FIG. 6**

## RÉFÉRENCES CITÉES DANS LA DESCRIPTION

**Documents brevets cités dans la description**

- US 5388591 A

**Littérature non-brevet citée dans la description**

- **SCHUMANN et al.** Time-frequency analysis of postural sway. *J. Biomechanics,* 1995, vol. 28 (5), 603-607
- **KIRCHNER et al.** Evaluation of the temporal structure of postural sway fluctuations based on a comprehensive set of analysis tools. *Physica A,* 2012, vol. 391, 4692-4703
- **AUDIFFREN et al.** A non linear scoring approach for evaluating balance : classification of elderly as fallers and non-fallers. *PLoS ONE,* 09 Décembre 2016, vol. 11 (12
- **BENDA, B.J. et al.** Biomechanical relationship between center of gravity and center of pressure during standing. *Rehabilitation Engineering, IEEE Transactions on,* 1994, vol. 2, 3-10